(19) Europäisches Patentamt
European Patent Office
Office européen des brevets

(11) Veröffentlichungsnummer: **0 405 440 A1**

(12) # EUROPÄISCHE PATENTANMELDUNG

(21) Anmeldenummer: **90112118.6**

(22) Anmeldetag: **26.06.90**

(51) Int. Cl.5: **C07D 401/12**, C07D 403/12, C07D 405/12, C07D 409/12, C07D 413/12, C07D 417/12, A01N 43/40, A01N 43/84

(30) Priorität: **29.06.89 CH 2411/89**

(43) Veröffentlichungstag der Anmeldung: **02.01.91 Patentblatt 91/01**

(84) Benannte Vertragsstaaten: **AT BE CH DE DK ES FR GB GR IT LI LU NL SE**

(71) Anmelder: **CIBA-GEIGY AG**
**Klybeckstrasse 141**
**CH-4002 Basel(CH)**

(72) Erfinder: **Riebli, Peter**
**Bienenheim**
**CH-6074 Giswil(CH)**

(74) Vertreter: **Zumstein, Fritz, Dr. et al**
**Bräuhausstrasse 4**
**D-8000 München 2(DE)**

(54) **Mikrobizide.**

(57) Verbindungen der Formel I und ihre Säureadditionssalze stellen wertvolle Mikrobizide dar und lassen sich in Form von Mitteln im Pflanzenschutz zur Verhütung und Bekämpfung von Mikroorganismen-Befall einsetzen.

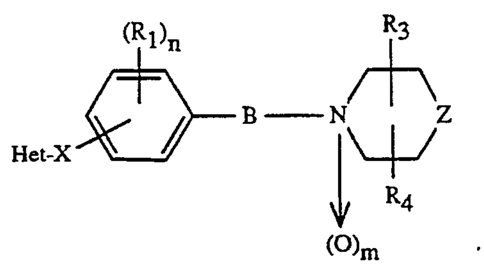

(I)

In der Formel bedeuten:
X Sauerstoff oder Schwefel,
Het einen 5- oder 6-gliedrigen Heterocyclus, mit einem bis drei gleichen oder verschiedenen Heteroatomen ausgewählt aus Stickstoff, Sauerstoff und Schwefel, der unsubstituiert ist oder mit gleichen oder verschiedenen Substituenten ausgewählt aus Halogen, Cyano, Nitro, $C_1$-$C_4$-Alkyl, $C_3$-$C_6$-Cycloalkyl, $C_1$-$C_4$-Alkoxy, $C_1$-$C_3$-Haloalkoxy und Trifluormethyl ein-, zwei- oder dreifach substituiert ist,
B $-CH_2-CH(R_2)-CH_2-$ oder $-CH=C(R_2)-CH_2-$ oder $-CH_2-C(R_2)=CH-$,
$R_1$ Halogen, $C_1$-$C_4$-Alkyl, $C_1$-$C_4$-Alkoxy,
n 0 bis 2,
m 0 oder 1,
$R_2$ Wasserstoff, $C_1$-$C_4$-Alkyl,
$R_3$ Wasserstoff, $C_1$-$C_3$-Alkyl,

EP 0 405 440 A1

$R_4$ Wasserstoff, Hydroxy, $C_1$-$C_3$-Alkoxy oder aber $C_1$-$C_4$-Alkyl, das unsubstituiert oder durch Hydroxy oder $C_1$-$C_3$-Alkoxy substituiert ist,
Z Sauerstoff, Schwefel oder $-CH_2-$.

## MIKROBIZIDE

Die vorliegende Erfindung betrifft aminoaliphatische Heterocyclyl-Phenylether der nachstehenden Formel I sowie deren Säureadditionssalze. Die Erfindung betrifft ferner die Herstellung dieser Substanzen sowie mikrobizide Mittel, die als Wirkstoff mindestens eine dieser Verbindungen enthalten. Die Erfindung betrifft auch die Verwendung der Wirkstoffe zur Bekämpfung schädlicher Mikroorganismen, insbesondere pflanzenpathogener Fungi

Die erfindungsgemässen Verbindungen haben die Formel

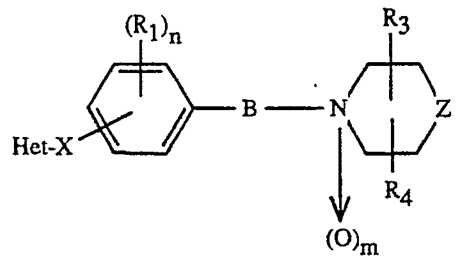

(I)

worin bedeuten:

X Sauerstoff oder Schwefel,

Het einen 5- oder 6-gliedrigen Heterocyclus, mit einem bis drei gleichen oder verschiedenen Heteroatomen ausgewählt aus Stickstoff, Sauerstoff und Schwefel, der unsubstituiert ist oder mit gleichen oder verschiedenen Substituenten ausgewählt aus Halogen, Cyano, Nitro, $C_1$-$C_4$-Alkyl, $C_3$-$C_6$-Cycloalkyl, $C_1$-$C_4$-Alkoxy, $C_1$-$C_3$-Haloalkoxy und Trifluormethyl ein-, zwei- oder dreifach substituiert ist,

B $-CH_2-CH(R_2)-CH_2-$ oder $-CH=C(R_2)-CH_2-$ oder $-CH_2-C(R_2)=CH-$,

$R_1$ Halogen, $C_1$-$C_4$-Alkyl, $C_1$-$C_4$-Alkoxy,

n 0 bis 2,

m 0 oder 1,

$R_2$ Wasserstoff, $C_1$-$C_4$-Alkyl,

$R_3$ Wasserstoff, $C_1$-$C_3$-Alkyl,

$R_4$ Wasserstoff, Hydroxy, $C_1$-$C_3$-Alkoxy oder aber $C_1$-$C_4$-Alkyl, das unsubstituiert oder durch Hydroxy oder $C_1$-$C_3$-Alkoxy substituiert ist,

Z Sauerstoff, Schwefel oder $-CH_2$;

unter Einschluss der Säureadditionssalze dieser Verbindungen.

Unter dem Begriff Alkyl selbst oder als Bestandteil eines anderen Substituenten wie Alkoxy oder Haloalkoxy etc., sind je nach Zahl der angegebenen Kohlenstoffatome beispielsweise die folgenden geradkettigen oder verzweigten Gruppen zu verstehen: Methyl, Ethyl, Propyl, Butyl sowie ihre Isomeren, wie z.B. Isopropyl, Isobutyl, tert.-Butyl usw. Halogen und Halo stehen für Fluor, Chlor, Brom oder Jod. Haloalkoxy steht somit für einen einfach bis perhalogenierten Alkoxyrest, wie z.B. $OCHCl_2$, $OCH_2F$, $OCCl_3$, $OCH_2Cl$, $OCHF_2$, $OCHFCH_3$, $OCH_2CH_2Br$, $OC_2Cl_5$, $OCH_2Br$, $OCHBrCl$ usw. $C_3$-$C_6$-Cycloalkyl steht wahlweise für Cyclopropyl, Cyclobutyl, Cyclopentyl oder Cyclohexyl.

Als 5- oder 6-gliedriger Heterocyclus mit einem, zwei oder drei gleichen oder verschiedenen Heteroatomen N, O und/oder S kommen z.B. in Frage: Pyridin, Pyrimidin, Pyrazin, Pyridazin, Triazin, Thiazol, Oxazol, Thiadiazol, Oxadiazol, Triazol, Imidazol, Furan, Thiophen, sowie entsprechende hydrierte oder teilhydrierte Heterocyclen wie z.B. Piperidin, Morpholin, Thiomorpholin, Pyran, Tetrahydrofuran und andere. Diese Aufzählung ist nicht limitierend.

Beispiele salzbildender Säuren sind anorganische Säuren: Halogenwasserstoffsäure wie Fluorwasserstoffsäure, Chlorwassertoffsäure, Bromwasserstoffsäure oder Jodwasserstoffsäure sowie Schwefelsäure, Phosphorsäure, Phosphorige Säure, Salpetersäure und organische Säuren wie Essigsäure, Trifluoressigsäure, Trichloressigsäure, Propionsäure, Weinsäure, Glykolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Salicylsäure, p-Aminosalicylsäure, 2-Phenoxybenzoesäure oder 2-Acetoxybenzoesäure. Diese Säuren werden nach an sich bekannten Methoden an die freien Verbindungen der Formel I addiert.

Die Verbindungen der Formel I sind bei Raumtemperatur stabil. Sie lassen sich auf dem Agrarsektor

oder verwandten Gebieten präventiv und kurativ zur Bekämpfung von pflanzenschädigenden Mikroorganismen einsetzen. Die erfindungsgemässen Wirkstoffe der Formel I zeichnen sich in weiten Anwendungskonzentrationen durch eine sehr gute fungizide Wirkung und problemlose Anwendung aus.

Aufgrund ihrer ausgeprägten mikrobiziden Wirkung sind folgende Substanzgruppen unter Einschluss ihrer Säureadditionssalze bevorzugt.

Verbindungen der Untergruppe der Formel I, worin der 5- oder 6-gliedrige Heterocyclus Het mindestens ein N-Atom enthält, $R_1$ Halogen, Methyl oder Methoxy bedeutet, n 0 oder 1 ist, m 0 ist und $R_2$ und $R_3$ unabhängig voneinander Wasserstoff oder Methyl bedeuten. (Untergruppe 1a)

Innerhalb der Untergruppe 1a sind jene Verbindungen bevorzugt, worin X Sauerstoff bedeutet und R4 Wasserstoff, Hydroxy, Methyl, Methoxy, Hydroxymethyl, Hydroxyethyl, Methoxymethyl oder Methoxyethyl darstellt. (Untergruppe 1aa)

Eine wichtige Verbindungsklasse sind jene der Untergruppe 1aa, worin Het einen unsubstituierten oder substituierten Pyridylrest bedeutet. (Untergruppe 1b)

Unter diesen Verbindungen sind jene bevorzugt, worin B die Kette $-CH_2-CH(CH_3)-CH_2-$oder $-CH=C-(CH_3)-CH_2-$ bedeutet, und der Pyridylrest unsubstituiert oder durch maximal zwei der Substituenten Halogen, $NO_2$, $-OCHF_2$ und $CF_3$ gleich oder verschieden substituiert ist. (Untergruppe 1bb)

Unter den letztgenannten Verbindungen sind jene bevorzugt, worin Z Sauerstoff oder $-CH_2-$ darstellt und $R_3$ und $R_4$ die beiden zu Z benachbarten Stellungen einnehmen und beide die gleiche Bedeutung Wasserstoff oder Methyl haben.

Eine andere wichtige Verbindungsklasse ist jene der Untergruppe 1aa, worin Het einen unsubstituierten oder substituierten Pyrimidinylrest bedeuteL (Untergruppe 1c)

Unter diesen Verbindungen 1c sind jene bevorzugt, worin B die Kette $-CH_2-CH(CH_3)-CH_2-$ oder $-CH=C(CH_3)-CH_2-$ bedeutet, und der Pyrimidinylrest unsubstituiert ist oder durch Halogen, $C_1$-$C_4$-Alkyl, Cyclopropyl gleich oder verschieden ein- bis dreifach substituiert ist. (Untergruppe 1cc)

Unter den letztgenannten Verbindungen sind jene bevorzugt, worin Z Sauerstoff oder $-CH_2-$ darstellt und $R_3$ und $R_4$ die beiden zu Z benachbarten Stellungen einnehmen und beide die gleiche Bedeutung Wasserstoff oder Methyl haben.

Eine andere wichtige Verbindungsklasse ist jene der Untergruppe 1aa, worin Het einen unsubstituierten oder substituierten Pyrazinylrest bedeutet. (Untergruppe 1d)

Unter diesen Verbindungen 1d sind jene bevorzugt, worin B die Kette $-CH_2-CH(CH_3)-CH_2-$ oder $-CH=C(CH_3)-CH_2-$ bedeutet und der Pyrazinylrest unsubstituiert oder durch $C_1$-$C_4$-Alkyl und/oder Halogen maximal zweifach substituiert ist. (Untergruppe 1dd)

Unter den letztgenannten Verbindungen sind solche bevorzugt, worin Z Sauerstoff oder $-CH_2-$ darstellt und $R_3$ und $R_4$ die beiden zu Z benachbarten Stellungen einnehmen und beide die gleiche Bedeutung Wasserstoff oder Methyl haben.

Eine weitere wichtige Verbindungsklasse ist jene der Untergruppe 1aa, worin Het einen unsubstituierten oder substituierten 1,3,5-Triazinylrest bedeutet. (Untergruppe 1e)

Unter den Verbindungen 1e sind jene bevorzugt, worin B die Kette $-CH_2-CH(CH_3)-CH_2-$oder $-CH=C-(CH_3)-CH_2-$ bedeutet und der 1,3,5-Triazinylrest unsubstituiert, alkyl-und/oder halogensubstituiert ist. (Untergruppe 1ee)

Unter den letztgenannten Verbindungen sind solche bevorzugt, worin Z Sauerstoff oder $-CH_2-$ darstellt und $R_3$ und $R_4$ die beiden zu Z benachbarten Stellungen einnehmen und beide die gleiche Bedeutung Wasserstoff oder Methyl haben.

Eine andere wichtige Verbindungsklasse innerhalb der Untergruppe 1aa sind solche, worin Het einen unsubstituierten oder substituierten Thiazolylrest bedeutet. (Untergruppe 1f)

Unter den Verbindungen 1f sind jene bevorzugt, worin B die Kette $-CH_2-CH(CH_3)-CH_2-$oder $-CH=C-(CH_3)-CH_2-$ bedeutet, und der Thiazolylrest unsubstituiert oder durch $NO_2$ oder Halogen monosubstituiert ist. (Untergruppe 1ff)

Unter den letztgenannten Verbindungen sind solche bevorzugt, worin Z Sauerstoff oder $-CH_2-$ darstellt und $R_3$ und $R_4$ die beiden zu Z benachbarten Stellungen einnehmen und beide die gleiche Bedeutung Wasserstoff oder Methyl haben.

Eine andere wichtige Verbindungsklasse innerhalb der Untergruppe 1aa sind solche, worin Het einen Oxazolylrest bedeutet (= Untergruppe 1g), vor allem solche, worin Z Sauerstoff oder $-CH_2-$ darstellt und $R_3$ und $R_4$ die beiden zu Z benachbarten Stellungen einnehmen und beide die gleiche Bedeutung Wasserstoff oder Methyl haben.

Eine weitere wichtige Verbindungsklasse innerhalb der Untergruppe 1aa sind Verbindungen, worin Het einen unsubstituierten oder durch $C_1$-$C_4$-Alkyl substituierten 1,2,4-Triazolylrest bedeutet (= Untergruppe 1h), vor allem solche, worin Z Sauerstoff oder $-CH_2-$ darstellt und $R_3$ und $R_4$ die beiden zu Z benachbarten

Stellungen einnehmen und beide die gleiche Bedeutung Wasserstoff oder Methyl haben.

Eine weitere wichtige Verbindungsklasse innerhalb der Untergruppe 1aa sind Verbindungen, worin Het einen unsubstituierten oder durch $C_1$-$C_4$-Alkyl substituierten 1,2,4-Imidazolylrest bedeutet (= Untergruppe 1i), vor allem solche, worin Z Sauerstoff oder $-CH_2-$ darstellt und $R_3$ und $R_4$ die beiden zu Z benachbarten Stellungen einnehmen und beide die gleiche Bedeutung Wasserstoff oder Methyl haben.

Eine weitere wichtige Verbindungsklasse innerhalb der Untergruppe 1aa sind Verbindungen, worin Het einen Oxadiazolrest bedeutet (= Untergruppe 1j), vor allem solche, worin Z Sauerstoff oder $-CH_2-$ darstellt und $R_3$ und $R_4$ die beiden zu Z benachbarten Stellungen einnehmen und beide die gleiche Bedeutung Wasserstoff oder Methyl haben.

Eine weitere wichtige Verbindungsklasse innerhalb der Untergruppe 1aa sind Verbindungen, worin Het einen Thiadiazolrest bedeutet (= Untergruppe 1k), vor allem solche, worin Z Sauerstoff oder $-CH_2-$ darstellt und $R_3$ und $R_4$ die beiden zu Z benachbarten Stellungen einnehmen und beide die gleiche Bedeutung Wasserstoff oder Methyl haben.

Eine weitere wichtige Untergruppe von Verbindungen sind jene der Formel I, worin Het einen unsubstituierten oder durch Halogen oder $C_1$-$C_4$Alkyl gleich oder verschieden maximal zweifach substituierten Thienylrest bedeutet. (Untergruppe 1m)

Unter den Verbindungen 1m sind jene bevorzugt, worin X Sauerstoff bedeutet, n 0 oder 1 ist, m 0 ist, $R_1$ Halogen, Methyl oder Methoxy darstellt, $R_2$ Wasserstoff oder Methyl und $R_3$ Wasserstoff oder Methyl bedeuten, und $R_4$ Wasserstoff, Methyl, Hydroxy, Methoxy, Hydroxymethyl oder Methoxymethyl darstellt. (Untergruppe 1mm)

Unter den letztgenannten Verbindungen sind solche bevorzugt, worin Z Sauerstoff oder $-CH_2-$ darstellt und $R_3$ und $R_4$ die beiden zu Z benachbarten Stellungen einnehmen und beide die gleiche Bedeutung Wasserstoff oder Methyl haben.

Die neuen Verbindungen der Formel I lassen sich herstellen, sofern $m = 0$ ist, durch

a) Umsetzung eines Amins der Formel II

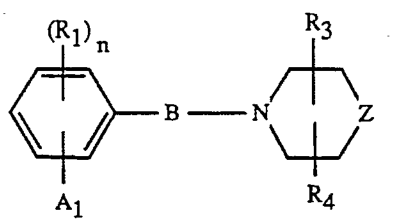

(II)

mit einem Heterocyclus der Formel III

Het-$A_2$ (III)

worin einer der Reste $A_1$ und $A_2$ eine nukleofuge Abgangsgruppe und der andere die Gruppe -XMe bedeutet, in der Me Wasserstoff oder vorzugsweise ein Metallkation ist.

Bevorzugt ist $A_1$ = XMe. Metallkationen Me sind beispielsweise Alkalimetall-, z.B. Lithium-, Natrium- oder Kaliumkationen, oder Erdalkalimetall, z.B. Magnesium-, Calcium-, Strontium- oder Bariumkationen.

Nukleofuge Abgangsgruppen sind beispielsweise reaktionsfähige veresterte Hydroxygruppen, die mit einer Halogenwasserstoffsäure, z.B. mit Fluor-, Chlor-, Brom- oder Jodwasserstoffsäure, oder die mit einer Niederalkansulfonsäure, oder die mit einer gegebenenfalls substituierten Benzol- oder Halogensulfonsäure verestert sind; beispielsweise mit Methan-, Ethan-, Benzol-, p-Toluol- oder Fluorsulfonsäure veresterte Hydroxygruppen.

Verbindungen der Formel II, bei denen $A_1$ OH oder SH bedeutet, sind teilweise bekannt, oder können nach an sich bekannten Verfahren hergestellt werden (z.B. EP-A-262 870).

Die Substituenten $R_1$, $R_3$, $R_4$, B, X und Z sowie n haben die unter Formel I angegebenen Bedeutungen.

Die Reaktion wird bevorzugt in einem relativ polaren, jedoch reaktionsinerten organischen Lösungsmittel durchgeführt, z.B. N,N-Dimethylformamid, N,N-Dimethalyetamid, Dimethylsulfoxid, Acetonitril, Benzonitril und anderen. Derartige Lösungsmittel können in Kombination mit anderen reaktionsinerten Lösungsmitteln, wie aliphatischen oder aromatischen Kohlenwasserstoffen, z.B. Benzol, Toluol, Xylol, Hexan, Petrolether, Chlorbenzol, Nitrobenzol u.a. verwendet werden.

Erhöhte Temperaturen von 0 bis 220 °C, bevorzugt 120-170 °C, sind vorteilhaft. Zweckmässig wird das Reaktionsgemisch unter Rückfluss erhitzt.

Die Verbindungen der Formel I mit $m = 0$ lassen sich ferner herstellen durch

b) Kondensation eines heterocyclischen Diphenylethers der Formel IV

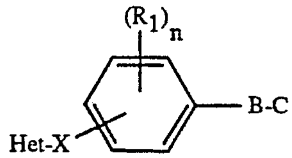

(IV)

worin C eine nukleofuge Abgangsgruppe darstellt, mit einem Amin der Formel V

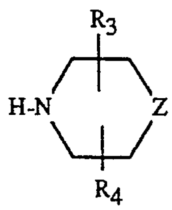

(V)

Die Reaktion kann ohne Lösungsmittel oder in Gegenwart von Lösungsmitteln bei 0° bis 120°C durchgeführt werden.

Als Lösungsmittel können bei Bedarf Ether (z.B. Diethylether, Tetrahydrofuran, Dioxane), Alkohole (z.B. Ethylenglykol, Methanol, Ethanol), N,N-Dimethylformamid, N,N-Dimethylacetamid, Dimethylsulfoxid etc. verwendet werden, aber auch überschüssiges Amin der Formel V.

c) Verbindungen der Formel I, worin m = 0 und B die Kette $-CH_2-C(R_2)=CH-$ oder $-CH_2-CH(R_2)-CH_2-$ bedeuten, können auch erhalten werden, durch Reaktion eines Aldehyds der Formel VI

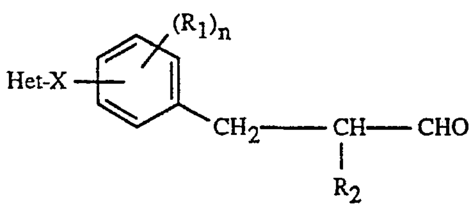

(VI)

mit einem Amin der Formel V zu einer Verbindung der Formel VII

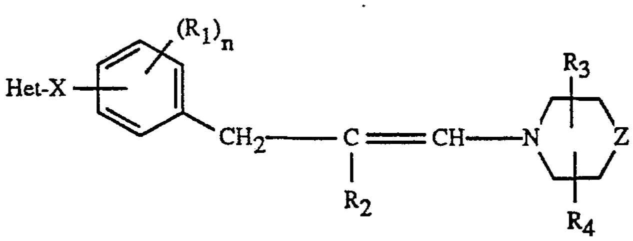

(VII)

im Umfang der Formel I und, sofern gewünscht, anschliessend Hydrierung der Doppelbindung.

Die Reaktion eines Aldehyds der Formel VI mit einem Amin der Formel V wird bei Temperaturen von -25°C bis +300°C, bevorzugt bei +10° bis 200°C, durchgeführt, zweckmässig unter Verwendung eines inerten Lösungsmittels. Als solches kommen aliphatische und aromatische Kohlenwasserstoffe, auch chlorierte Kohlenwasserstoffe, in Frage, z.B. Petrolether, Hexan, Toluol, Xylol, Benzol, Chlorbenzol, Methylenchlorid, Chloroform, Dichlorethan, Dibrommethan; ferner Ether wie Diethylether, Methyl-tert.Butylether, Dioxan, Tetrahydrofuran (THF); ferner Alkohole wie Methanol, Ethanol, Ethylenglykol, Glycerin oder Ester wie Methylacetat oder Ethylacetat.

Die Anwesenheit wasserentziehender Mittel ist vorteilhaft. Es lassen sich beispielsweise wasserfreies $Na_2SO_4$, $MgSO_4$, $CaCl_2$, Silica Gel, $Al_2O_3$ oder Molekularsieb verwenden.

Saure Katalysatoren beschleunigen die Reaktion, z.B. Chlorwasserstoff, Bromwasserstoff, Schwefelsäure, Salpetersäure, Essigsäure, Benzolsulfonsäure, p-Toluolsulfonsäure, Methansulfonsäure u.a.

Sollen die ungesättigten Enamine der Formel VII (im Formelumfang I vorliegender Erfindung) zu

gesättigten Verbindungen hydriert werden, so lassen sich für diese Reaktion Metallhydride als Reduktionsmittel verwenden, z.B. Natrium-, Kalium- oder Lithiumborhydrid.

Als geeignete Lösungsmittel oder Lösungsmittelgemische untereinander lassen sich die bereits genannten Alkohole, Ether, Ester und aromatischen Kohlenwasserstoffe, ferner auch Essigsäure oder Wasser einsetzen.

Als Reduktionsmittel kommen auch Aluminiumhydride wie Lithiumaluminiumhydrid oder Aluminiumdiisobutylhydrid in Frage, die vorteilhaft in den genannten Ethern oder Kohlenwasserstoffen oder Gemischen dieser Lösungsmittel verwendet werden.

Die Hydrierung erfolgt im Temperaturbereich von -60° bis +200°C, vorzugsweise -20° bis +120°C.

Als Reduktionsmittel kommt auch Ameisensäure in Frage, und die Reaktion wird vorzugsweise in Abwesenheit eines Lösungsmittels durchgeführt. Die Ameisensäure wird zum Enamin bei einer Temperatur von 0° bis 100°C, vorzugsweise 50-70°C, zugetropft, notwendigenfalls unter Kühlung.

Verbindungen der Formel VII können auch katalytisch hydriert werden. Als Katalysatoren eignen sich besonders Edelmetallkatalysatoren wie beispielsweise Platin, Palladium-gegebenenfalls auf Kohle niedergeschlagen - sowie Raney-Nickel. Bevorzugt ist Palladium auf Kohle.

Geeignete inerte Lösungsmittel sind Kohlenwasserstoffe wie Benzol, Toluol oder Xylol sowie Alkohole wie Methanol oder Äthanol. Bevorzugt ist Toluol. Als Reaktionstemperatur wird vorteilhaft ein Intervall zwischen 0° und +50°C, bevorzugt Raumtemperatur, gewählt.

Aus den vorstehend gemäss a), b) oder c) gewonnenen Verbindungen der Formel I mit $m=0$ lassen sich, sofern gewünscht, durch Oxidation die entsprechenden N-Oxide ($m=1$) der Formel I gewinnen.

Als Oxidationsmittel können u.a. Wasserstoffperoxid, Perbenzoesäure, Peressigsäure, m-Chlorperbenzoesäure verwendet werden.

Die Reaktion wird in einem Temperaturbereich von -20° bis 200°C, bevorzugt 0 bis 120°C, in einem inerten Lösungsmittel durchgeführt. Beispiele von Lösungsmitteln sind chlorierte Kohlenwasserstoffe (z.B. Chloroform, Dichlormethan, Dichlorethan), Alkohole (z.B. Methanol, Ethanol, Ethylenglykol), Ether (z.B. Diethylether, THF, Dioxane), N,N-Dimethylformamid, N,N-Dimethylacetamid, Dimethylsulfoxid etc. Diese Lösungsmittel können alleine oder in Kombination verwendet werden.

Sämtliche der vorstehend beschriebenen Verfahren a), b) und c) sind samt ihren Varianten und dem gegebenenfalls angewendeten Reaktionsschritt der Nachoxidation (zur Erzielung von N-Oxiden) Gegenstand vorliegender Erfindung.

Die Verbindungen der Formel IV und VI sind neu, können jedoch nach den üblichen Methoden der präparativen organischen Chemie hergestellt werden, so z.B. analog den in der DE-OS 27 52 135 beschriebenen Verfahren.

Die Propan-Derivate der Formel I besitzen in der 2-Stellung, sofern $R_2$ Alkyl bedeutet, stets ein asymmetrisches *C-Atom und können daher in zwei enantiomeren Formen vorliegen. Im allgemeinen entsteht bei der Herstellung dieser Substanzen ein Gemisch beider Enantiomeren. Dieses lässt sich auf übliche Weise, z.B. durch fraktionierte Kristallisation von Salzen mit optisch aktiven, starken Säuren, in die reinen optischen Antipoden aufspalten. Beide Enantiomeren können unterschiedliche biologische Aktivitäten aufweisen. Sobald infolge einer Doppelbindung in der 2-Stellung cis/trans-Isomerie auftritt, gelten die gleichen Überlegungen.

Die vorliegende Erfindung betrifft alle reinen Enantiomeren bzw. Diastereomeren und deren Gemische untereinander.

Es wurde überraschend festgestellt, dass Verbindungen der Formel I ein für praktische Bedürfnisse sehr günstiges Mikrobizid-Spektrum gegen Pilze und Bakterien aufweisen. Sie besitzen sehr vorteilhafte kurative, präventive und systemische Eigenschaften und lassen sich zumSchutz von Kulturpflanzen einsetzen. Mit den Wirkstoffen der Formel I können Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben.

Die Wirkstoffe sind gegen die den folgenden Klassen angehörenden phytopathogenen Pilze wirksam: Ascomyceten (z.B. Venturia, Podosphaera, Erysiphe, Monilinia, Pyrenophora, Uncinula); Basidiomyceten (z.B. die Gattungen Hemileia, Rhizoctonia, Puccinia); Fungi imperfecti (z.B. Botrytis, Helminthosporium, Fusarium, Septoria, Cercospora, Colletotrichum, Rhynchosporium, Pseudocercosporella und Alternaria). Überdies wirden die Verbindungen der Formel I systemisch. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden. Die erfindungsgemässen Wirkstoffe zeichnen sich durch besonders gute Pflanzenverträglichkeit aus.

Die Erfindung betrifft somit auch mikrobizide Mittel sowie die Verwendung der Verbindungen der

Formel I zur Bekämpfung pathogener Mikroorganismen, insbesondere pflanzenschädigender Pilze bzw. die präventive Verhütung eines Befalls an Pflanzen.

Als Zielkulturen für die hierin offenbarten Indikationsgebiete gelten im Rahmen dieser Erfindung z.B. folgende Pflanzenarten: Getreide: (Weizen, Gerste, Roggen, Hafer, Reis, Sorghum und Verwandte); Rüben: (Sucker und Futterrüben); Kern-, Stein- und Beerenobst: (Äpfel, Birnen, Pflaumen, Pfirsiche, Mandeln, Kirschen, Erdbeeren, Himbeeren und Brombeeren); Hülsenfrüchte: (Bohnen, Linsen, Erbsen, Soja); Ölkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Blumen, Sträucher, Laubbäume und Nadelbäume). Diese Aufzählung stellt keine Limitierung dar.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Die Verbindungen der Formel I werden dabei in unveränderter Form oder vorzugsweise zusammen mit den in der Formulierungstechnik üblichen Hilfsmitteln eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren werden wie die Art der Mittel den angestrebten Sielen und den gegebenen Verhältnissen entsprechend gewählt. Im Agrarsektor liegen günstige Aufwandmengen im allgemeinen bei 50 g bis 5 kg Aktivsubstanz (AS) je ha; bevorzugt 100 g bis 2 kg AS/ha, insbesondere bei 150 g bis 800 g.

Als Lösungsmittel können in Frage kommen: Aromatische Kohlenwasserstoffe, bevorzugt die Fraktion $C_8$-$C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Besonders vorteilhafte, applikationsfördernde Zuschlagstoffe, die zu einer starken Reduktion der Aufwandmenge führen können, sind ferner natürliche (tierische oder pflanzliche) oder synthetische Phospholipide aus der Reihe der Kephaline und Lecithine, wie z.B. Phosphatidylethanolamin, Phosphatidylserin, Phosphatidylglycerin oder Lysolecithin.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Die in der Formuliervngstechnik gebräuchlichen Tenside sind u.a. in folgenden Publikationen beschrieben:

"Mc Cutcheon's Detergents and Emulsifiers Annual"
BC Publishing Corp., Ringwood New Jersey, 1981;
Helmut Stache "Tensid-Taschenbuch" Carl Hanser-Verlag
München/Wien 1981.
M. and J. Ash. "Encyclopedia of Surfactants", Vol. I-III, Chemical Publishing Co., New York, 1980-1981.

Die agrochemischen Zubereitungen enthalten in der Regel 0,1 bis 99 %, insbesondere 0,1 bis 95 % Wirkstoff der Formel I, 99,9 bis 1 %,, insbesondere 99,8 bis 5 % eines festen oder flüssigen Streckmittels und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Die nachfolgenden Beispiele dienen zur näheren Erläutetung der Erfindung, ohne dieselbe einzuschränken. Temperaturen sind in Celsiusgraden angegeben. Prozente und Teile beziehen sich auf das Gewicht.

Herstellungsbeispiele

Beispiel 1: Herstellung von

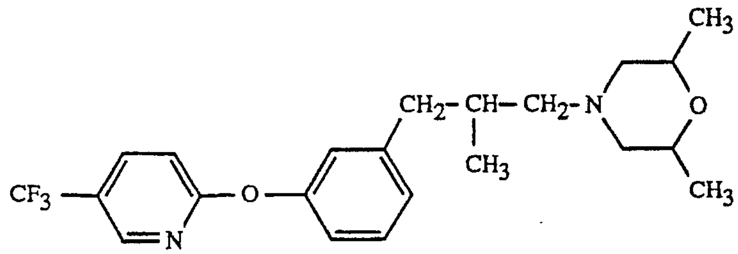

4-[3-{3-(5-Trifluormethyl-2-pyridyloxy)phenyl}-2-methylpropyl]-2,6-dimethylmorpholin

Zu einer Lösung von 3,6 g 4-[3-(3-Hydroxyphenyl)-2-methylpropyl]-2,6-dimethylmorpholin in 40 ml absolutem Methanol werden bei Raumtemperatur 2,7 g 30%iges Natriummethylat zugegeben. Das Gemisch wird eine halbe Stunde am Rückfluss erhitzt, dann wird Methanol am Rotationsverdampfer entfernt und das kristalline Natriumsalz mit 50 ml absolutem Dimethylformamid versetzt. Dazu werden 2,0 g 2-Chlor-5-trifluormethylpyridin und 0,3 g katalytisch wirkendes Kaliumjodid gegeben. Anschliessend wird 18 Stunden bei 100 °C gerührt, dann auf Raumtemperatur abgekühlt, das Reaktionsgemisch auf 150 ml Wasser gegossen und dreimal mit je 50 ml Ethylacetat extrahiert. Die vereinigten organischen Phasen werden zweimal mit je 100 ml Wasser gewaschen, über Natriumsulfat getrocknet, filtriert und vom Lösungsmittel durch Abdampfen befreit. Der braune ölige Rückstand wird zur Reinigung mit Hexan/Ethylacetat (2:1) über eine Kieselgelsäule chromatographiert. Die Titelverbindung wird als gelbes Oel erhalten; $n_D^{23}$ : 1,5012.

Beispiel 2: Herstellung von

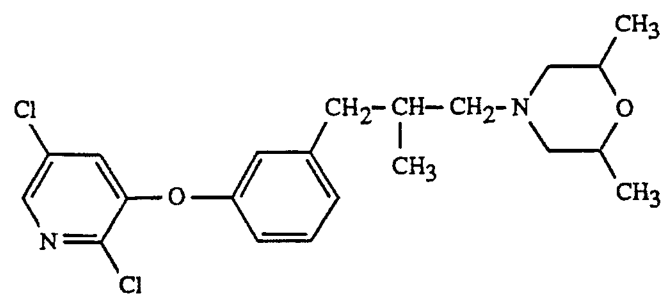

4-[3-{3-((2,5-Dichlor-3-pyridyloxy)phenyl}-2-methylpropyl]-2,6-dimethylmorpholin

Zu einer Lösung von 3,6 g 4-[3-(3-Hydroxyphenyl)-2-methylpropyl]-2,6-dimethylmorpholin in 40 ml absolutem Methanol werden 0,8 g festes Natriummethylat zugegeben und das Gemisch 30 min am Rückfluss erhitzt. Dann wird das Methanol am Rotationsverdampfer entfernt und das kristalline Natriumsalz mit 50 mi absolutem Dimethylacetamid, 2,5 g 2,5-Dichlor-3-fluorpyridin und 0,3 g Kaliumjodid versetzt. 20 Stunden wird dieses Gemisch bei 120 °C gerührt, dann auf Raumtemperatur abgekühlt und auf 250 ml Wasser gegossen. Nach dreimaliger Extraktion mit je 100 ml Ethylacetat werden die organischen Phasen vereinigt, 2x mit je 150 ml Wasser gewaschen, über Natriumsulfat getrocknet, filtriert und am Rotationsverdampfer vomLösungsmittel befreit. Der dunkle, ölige Rückstand wird zur Reinigung mittels Hexan-Ethylacetat (2:1) über eine Kieselgelsäule chromatographiert. Nach Verdampfen des Laufmittelgemisches wird ein gelbes Oel erhalten; $n_D^{23}$ : 1,5508.

Synthese von Zwischenprodukten

Beispiel 3: Herstellung von

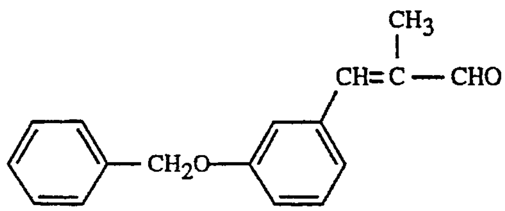

3-(3-Benzyloxyphenyl)-2-methacrolein

31,8 g 3-Benzyloxybenzaldehyd werden in 200 ml Methanol gelöst, mit 0,5 g pulverisiertem Kaliumhydroxid versetzt und auf 45° C erwärmt. Bei dieser Temperatur (± 5° C) werden innert 1 Stunde 26,0 g Propionaldehyd zugetropft und das Gemisch 4 Stunden bei 40-45° C gerührt. Anschliessend wird das Lösungsmittel am Rotationsverdampfer verdampft, der ölige Rückstand auf 500 ml Wasser gegossen, und 3x mit je 150 ml Diethylether extrahiert. Die organischen Phasen werden vereinigt, 2x mit je 200 ml Wasser gewaschen, über Natriumsulfat getrocknet, filtriert und eingeengt. Die Titelverbindung wird als gelbes Oel erhalten, $n_D^{20}$ : 1,6217.

Beispiel 4: Herstellung

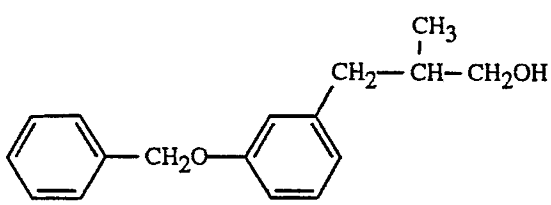

3-(3-Benzyloxyphenyl)-2-methyl-propanol

10,0 g Lithiumaluminiumhydrid werden unter Stickstoffbegasung in 350 ml absolutem Tetrahydrofuran am Rückfluss erhitzt. Dazu werden innert 2 Stunden bei Rückfluss 44,2 g des unter Beispiel 3 erhaltenen 3-(3-Benzyloxyphenyl)-2-methyl-acroleins, gelöst in 70 ml absolutem Tetrahydrofuran, zugetropft und weitere 3 Stunden am Rückfluss erhitzt. Anschliessend wird auf Raumtemperatur abgekühlt, und unter Kühlung werden vorsichtig 250 ml Eiswasser zugetropft. Dann wird die Reaktionsmischung mit 300 ml Diethylether versetzt und über Hyflo abgenutscht. Vom Filtrat wird die organische Phase abgetrennt und die wässrige Phase 2x mit je 250 ml Ether extrahiert. Die organischen Phasen werden vereinigt, 3x mit je 250 mi Wasser gewaschen, über Natriumsulfat getrocknet, filtriert und vom Lösungsmittel durch Abdampfen befreit. Sur Reinigung wird der ölige Rückstand mit Hexan/Ethylacetat (3:1) über eine Kieselgelsäule chromatographiert. Nach Verdampfen des Laufmittelgemisches wird ein gelbes Oel erhalten; $n_D^{22}$ : 1,5624.

Beispiel 5: Herstellung

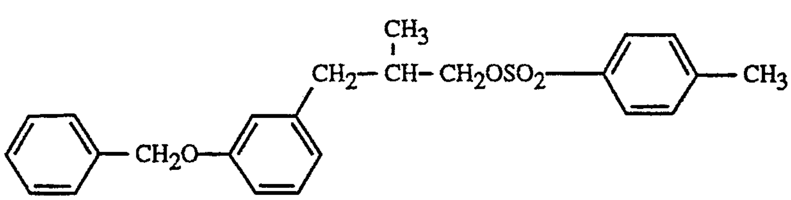

1-(p-Tosyloxy)-2-methyl-3-(3-benzyloxyphenyl)-propan

21,0 g 3-(3-Benzyloxyphenyl)-2-methyl-propanol werden in 80 ml Pyridin vorgelegt und auf -5 °C abgekühlt. Bei -5 °C bis 0 °C werden 17,2 g p-Toluolsulfonylchlorid portionenweise innert 1 Stunde zugegeben und das Gemisch 4 Stunden bei 0 ° gerührt. Anschliessend wird die Reaktionsmischung auf 300 ml Eiswasser gegossen und 3x mit je 200 ml Diethylether extrahiert. Die organischen Phasen werden vereinigt, 2x mit je 150 ml 2N Salzsäure und 3x mit je 200 ml Wasser gewaschen, über Natriumsulfat getrocknet und filtriert. Dann wird das Lösungsmittel verdampft.
Der ölige Rückstand wird mit Hexan/Ethylacetat (1:1) über eine Kieselgelsäure chromatographiert. Die Titelverbindung wird als gelbes Oel erhalten, $n_D^{22}$ : 1,5667.

Beispiel 6: Herstellung von

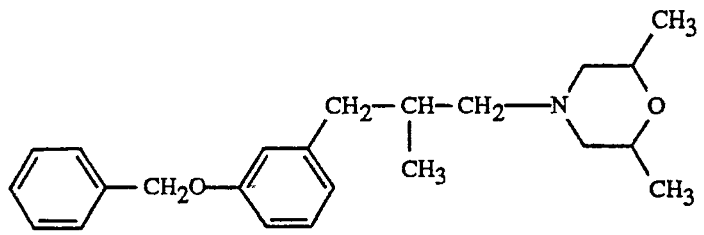

4-[3-(3-Benzyloxyphenyl)-2-methylpropyl]-2,6-dimethylmorpholin

8,7 g des in Beispiel 5 erhaltenen 1-(p-Tosyloxy)-2-methyl-3-(3-benzyloxyphenyl)-propans und 5,6g Dimethylmorpholin werden 1 1/2 Stunde bei 100 °C gerührt, dann auf Raumtemperatur abgekühlt und mit 50 ml Wasser und 50 ml Ethylacetat versetzt. Die oganische Phase wird abgetrennt, die wässrige Phase 2x mit je 50 ml Ethylacetat extrahiert. Die organischen Phasen werden vereinigt, 3x mit je 150 ml Wasser gewaschen, über Natriumsulfat getrocknet, filtriert und eingeengt.
Der ölige Rückstand wird zur Reinigung mit Hexan/Ethylacetat (2:1) über eine Kieselgelsäule chromatographiert. Nach Verdampfen des Laufmittelgemisches wird ein gelbes Oel erhalten; $n_D^{22}$ : 1,5381.

Beispiel 7: Herstellung von 4-[3-(3-Hydroxvphenyl)-2-methylpropyl]-2,6-dimethylmorpholin

10,0 g des in Beispiel 6 erhaltenen 4-[3-(3-Benzyloxyphenyl)-2-methylpropyl]-2,6-dimethylmorpholins werden in 70 ml Ethylacetat in Gegenwart von 1,0 Palladium-Kohle (5 %) bei Raumtemperatur innert 19 Stunden hydriert. Anschliessend wird der Katalysator durch Filtration abgetrennt und das Lösungsmittel am Rotationsverdampfer entfernt. Die Titelverbindung wird als viskoses Öl von gelber Farbe erhalten; $n_D^{55}$ : 1,5109.

Auf diese Weise oder nach Art einer der weiter oben angegebenen Methoden werden folgende Verbindungen hergestellt. Im folgenden bedeuten

Morph = 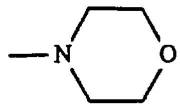

Pip. = 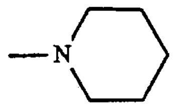

DMM = 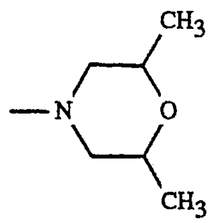

DMP = 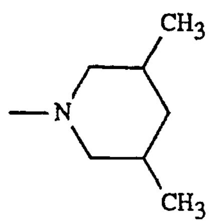 cis-DMP = 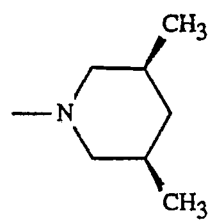

cis-DMM = 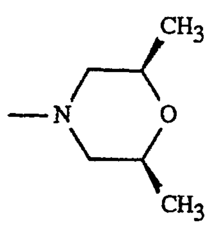 trans-DMM = 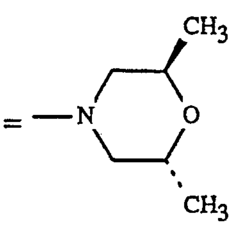

trans-DMP = 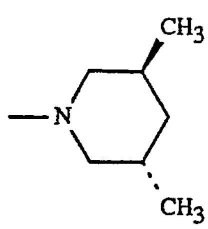

Tabelle 1

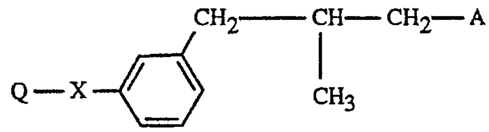

| Nr. | Q | A | X | phys. Daten |
|---|---|---|---|---|
| 1.1. | 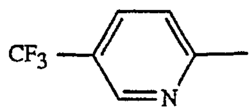 | DMM | O | $n_D^{23}$ : 1,5012 |
| 1.2.a | 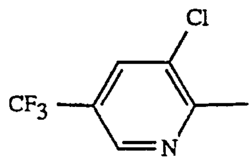 | cis-DMP | O | $n_D^{39}$ : 1,5083 |
| 1.2.b | | trans-DMP | | $n_D^{39}$ : 1,5088 |
| 1.3. | 2,5-Dichlor-3-pyridyl | Pip. | S | |
| 1.4. | 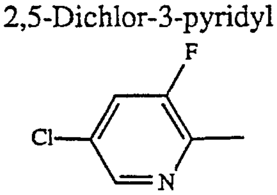 | Pip. | O | |
| 1.5. | 2-Thienyl | Morph | O | |
| 1.6. | 2-Pyridyl | DMM | O | |
| 1.7. | 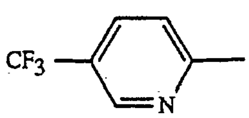 | Thiomorpholin | O | |
| 1.8. | 2-Pyrimidinyl | 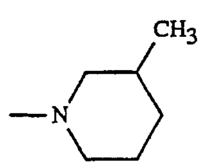 | O | |
| 1.9. | 2-Thiazolyl | Pip. | O | |

| Nr. | Q | A | X | phys. Daten |
|---|---|---|---|---|
| 1.10. | 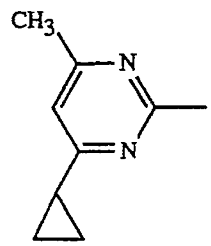 | DMM | O | |
| 1.11. | 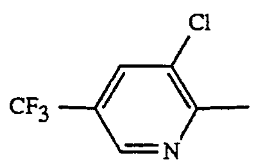 | Morph | O | |
| 1.12. | 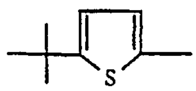 | DMM | O | |
| 1.13. | 2-Pyrazinyl | Pip. | O | |
| 1.14. | 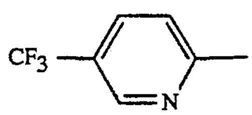 | cis-DMM | O | $n_D^{25}$ : 1,5066 |
| 1.15. | 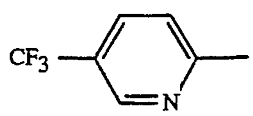 | trans-DMM | O | |
| 1.16. | 2,5-Dichlor-3-pyridyl | Pip. | O | |
| 1.17. | 3-Fluor-5-chlor-2-pyridyl | Morph | S | |
| 1.18. | 2-Pyridyl | Pip. | O | |
| 1.19. | 2-Thienyl | DMM | O | |

| Nr. | Q | A | X | phys. Daten |
|---|---|---|---|---|
| 1.20. | 3,5-Dichlor-2-pyridyl | DMM | S | |
| 1.21. | 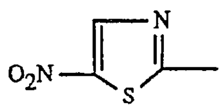 | Pip. | O | |
| 1.22. | 3-Fluor-5-chlor-2-pyridyl | DMM | O | $n_D^{25}$ : 1,5352 |
| 1.23. | 5-Chlor-2-pyridyl | Pip. | O | |
| 1.24. | 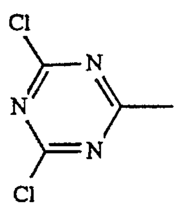 | DMM | O | |
| 1.25. | 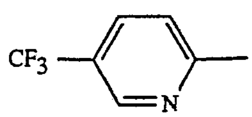 | Pip. | S | |
| 1.26. | 2-Pyrimidinyl | DMM | O | |
| 1.27. | 5-Brom-2pyridyl | Pip. | O | |
| 1.28. | 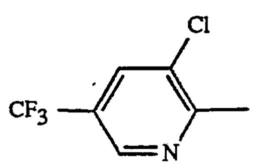 | cis-DMM | O | $n_D^{40}$ : 1,5090 [Nitrat: Smp. 148°C] |
| 1.29. | 2-Thienyl | Pip. | O | |

| Nr. | Q | A | X | phys. Daten |
|---|---|---|---|---|
| 1.30. | 3-Chlor-2-pyridyl | 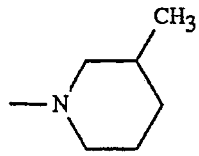 | O | |
| 1.31. | 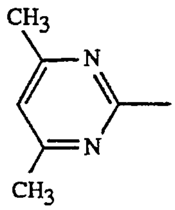 | Pip. | O | |
| 1.32. | 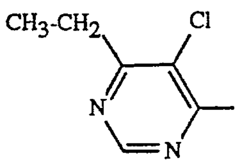 | DMM | O | |
| 1.33. | 2-Thiazolyl | DMM | O | |
| 1.34. | 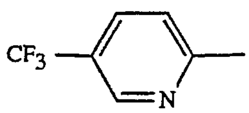 | DMP | O | |
| 1.35. | 3-Fluor-5-chlor-2-pyridyl | trans-DMM | O | |
| 1.36. | 3-Fluor-5-chlor-2-pyridyl | cis-DMM | O | $n_D^{40}$ : 1,5314 [Nitrat: Smp. 128°C] |
| 1.37. | 2-Pyrimidinyl | Pip. | O | |
| 1.38. | 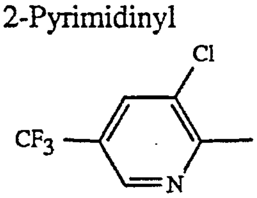 | Thiomorpolin | S | |

| Nr. | Q | A | X | phys. Daten |
|---|---|---|---|---|
| 1.39. | 2,5-Dichlor-3-pyridyl | DMM | O | $n_D^{25}$ : 1,5508 |
| 1.40. | 2-Pyridyl | Morph | O | |
| 1.41. | 3,5-Dichlor-2-pyridyl | Pip. | O | |
| 1.42. | 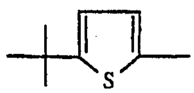 | Pip. | O | |
| 1.43. | 5-Chlor-2-pyridyl | Thiomorpholin | O | |
| 1.44. | 3-Chlor-2-pyridyl | Morph | O | |
| 1.45. | 2,5-Dichlor-3-pyridyl | DMP | O | |
| 1.46. | 2-Pyrazinyl | DMM | S | |
| 1.47. | 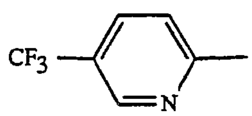 | 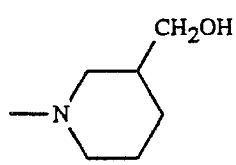 | O | |
| 1.48. | 2-Thienyl | cis-DMM | O | |
| 1.49. | 2-Thienyl | trans-DMM | O | |
| 1.50. | 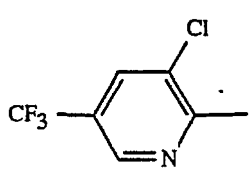 | DMM | O | |
| 1.51. | 5-Brom-2-pyridyl | DMM | O | $n_D^{42}$ : 1,5483 |

| Nr. | Q | A | X | phys. Daten |
|---|---|---|---|---|
| 1.52. | 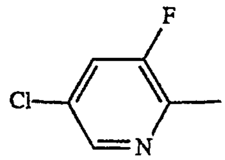 | Morph | O | |
| 1.53. | 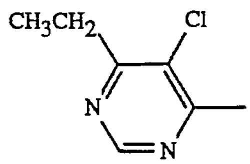 | Pip. | O | |
| 1.54. | 2-Pyridyl | DMM | S | |
| 1.55. | 5-Chlor-2-pyridyl | 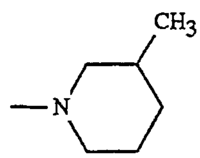 | O | |
| 1.56. | 4-Pyrimidinyl | DMM | O | |
| 1.57. | 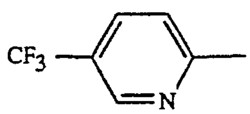 | cis-DMP | O | |
| 1.58. | 2-Pyrimidinyl | Morph | O | |
| 1.59. | 2,5-Dichlor-3-pyridyl | Morph | O | |
| 1.60. | 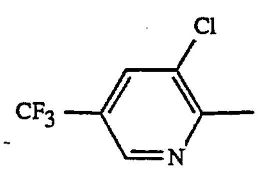 | 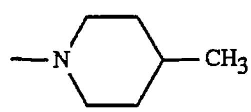 | O | |

| Nr. | Q | A | X | phys. Daten |
|---|---|---|---|---|
| 1.61. | 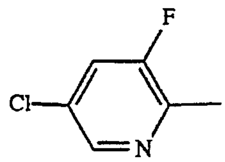 | DMP | O | $n_D^{35}$ : 1,5320 |
| 1.62. | 2-Thienyl | DMM | S | |
| 1.63. | 3,5-Dichlor-2-pyridyl | DMP | O | |
| 1.64. | 3-Chlor-2-pyridyl | DMM | O | |
| 1.65. | 5-Chlor-2-thienyl | Pip. | O | |
| 1.66. | 2-Pyrazinyl | DMM | O | |
| 1.67. | 5-Chlor-2-pyridyl | DMP | O | |
| 1.68. | 3-Thienyl | Pip. | O | |
| 1.69. | 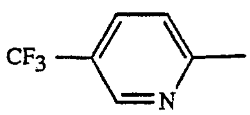 | Morph | O | |
| 1.70. | 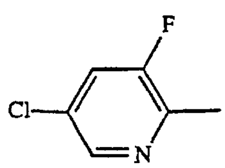 | 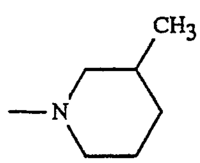 | O | |
| 1.71. | 3,5-Dichlor-2-pyridyl | Morph | O | |
| 1.72. | 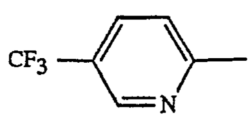 | Pip. | O | |

| Nr. | Q | A | X | phys. Daten |
|---|---|---|---|---|
| 1.73. | 2,5-Dichlor-3-pyridyl | cis-DMM | O | $n_D^{45}$ : 1,5428 |
| 1.74. | 2,5-Dichlor-3-pyridyl | trans-DMM | O | |
| 1.75. | 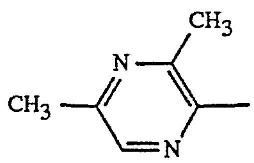 | Pip. | O | |
| 1.76. | 4,6-Dimethyl-2-pyrimidinyl | cis-DMM | O | $n_D^{34}$ : 1,5288 |
| 1.77. | 2-Thienyl | 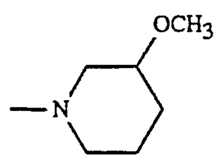 | O | |
| 1.78. | 2-Pyrimidinyl | cis-DMM | O | |
| 1.79. | 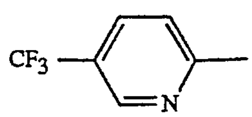 | DMM | S | |
| 1.80. | 5-Brom-2-pyridyl | DMP | O | |
| 1.81. | 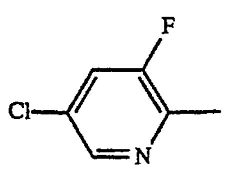 | 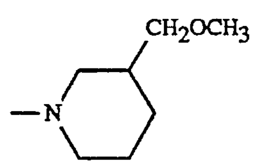 | O | |
| 1.82. | 3,5-Dichlor-2-pyridyl | DMM | O | $n_D^{40}$ : 1,5458 |

| Nr. | Q | A | X | phys. Daten |
|---|---|---|---|---|
| 1.83. | 2,5-Dichlor-3-pyridyl | 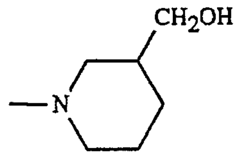 | O | |
| 1.84. | 5-Chlor-2-pyridyl | DMM | S | |
| 1.85. | 5-Chlor-2-thienyl | 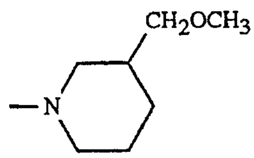 | O | |
| 1.86. | 3-Chlor-2-pyridyl | DMP | O | |
| 1.87. | 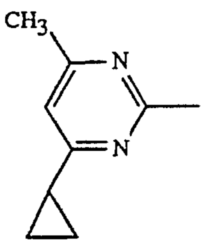 | Pip. | O | |
| 1.88. | 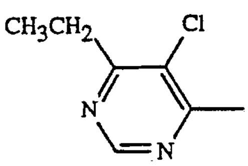 | DMP | O | |
| 1.89. | 6-Methoxy-2-pyridyl | Pip. | O | |
| 1.90. | 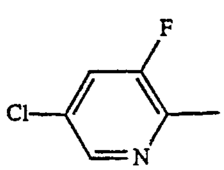 | cis-DMP | O | |

| Nr. | Q | A | X | phys. Daten |
|---|---|---|---|---|
| 1.91. | 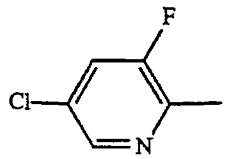 | 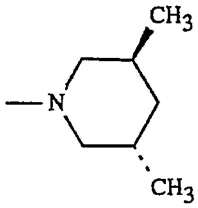 | O | |
| 1.92. | 3-Nitro-2-pyridyl | Pip. | O | |
| 1.93. | 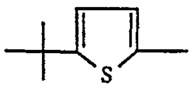 | DMM | S | |
| 1.94. | 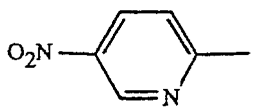 | Pip. | O | |
| 1.95. | 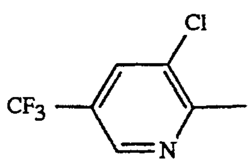 | Pip. | O | |
| 1.96. | 5-Chlor-2-thienyl | DMM | O | |
| 1.97. | 5-Brom-2-pyridyl | trans-DMM | O | |
| 1.98. | 4-Pyrimidinyl | Pip. | O | |
| 1.99. | 5-Nitro-2-thiazolyl | DMM | O | |
| 1.100. | 4-Pyridyl | Pip. | O | |

| Nr. | Q | A | X | phys. Daten |
|---|---|---|---|---|
| 1.101. | 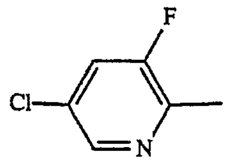 | 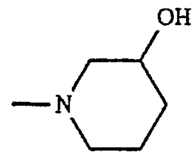 | O | |
| 1.102. | 5-Chlor-2-pyridyl | Morph | O | |
| 1.103. | 2-Pyrazinyl | DMP | O | |
| 1.104. | 3,5-Dichlor-2-pyridyl | trans-DMM | O | |
| 1.105. | 3,5-Dichlor-2-pyridyl | cis-DMM | O | $n_D^{39}$ : 1,5454 [Nitrat: Smp. 146°C] |
| 1.106. | 5-Chlor-2-thienyl | DMP | O | |
| 1.107. | 3-Thienyl | DMM | O | |
| 1.108. | 3-Chlor-2-pyridyl | Pip. | O | |
| 1.109. | 5-Nitro-2-pyridyl | DMM | O | |
| 1.110. | 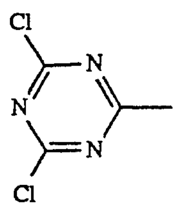 | Pip. | O | |
| 1.111. | 3-Pyridyl | DMM | O | |
| 1.112. | 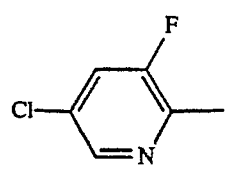 | DMM | S | |

| Nr. | Q | A | X | phys. Daten |
|---|---|---|---|---|
| 1.113. | 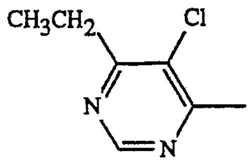 | cis-DMM | O | |
| 1.114. | 5-Chlor-2-pyridyl | DMM | O | |
| 1.115. | 4-Methyl-2-pyridyl | Pip. | O | |
| 1.116. | 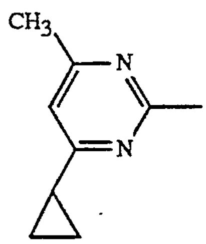 | Thiomorpholin | O | |
| 1.117 | 5-Chlor-2-thienyl | Morph | O | |
| 1.118. | 5-Brom-2-pyridyl | 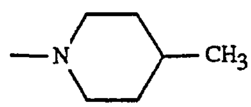 | O | |
| 1.119. | 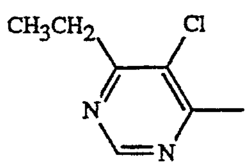 | cis-DMP | O | |
| 1.120. | 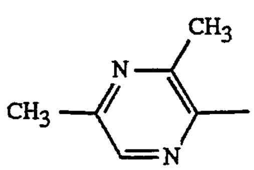 | DMM | O | |
| 1.121. | 4-Pyridyl | DMM | O | |

| Nr. | Q | A | X | phys. Daten |
|---|---|---|---|---|
| 1.122. | 3,5-Dichlor-2-pyridyl | 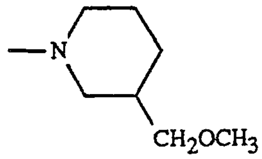 | O | |
| 1.123. | 4-Methyl-2-pyridyl | DMM | O | |
| 1.124. | 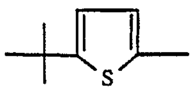 | cis-DMM | O | |
| 1.125. | 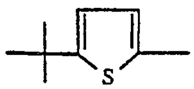 | trans-DMM | O | |
| 1.126. | 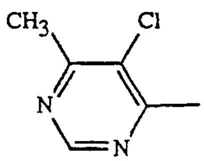 | Pip. | O | |
| 1.127. | 5-Chlor-2-thienyl | cis-DMP | O | |
| 1.128. | 5-Pyrimidinyl | DMM | O | |
| 1.129. | 3,5-Dichlor-2-pyridyl | 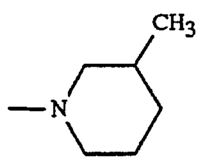 | O | |
| 1.130 | 5-Pyrimidinyl | Pip. | O | |
| 1.131. | 4-Pyrimidinyl | DMP | O | |

| Nr. | Q | A | X | phys. Daten |
|---|---|---|---|---|
| 1.132. | 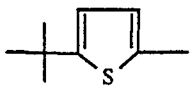 | 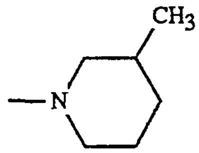 | O | |
| 1.133. | 3-Chlor-2-pyridyl | DMM | S | |
| 1.134. | 3-Nitro-2-pyridyl | DMM | O | |
| 1.135. | 3,5-Dichlor-2-pyridyl | cis-DMP | O | |
| 1.136. | 5-Chlor-2-thienyl | DMM | S | |
| 1.137. | 5-Chlor-2-pyridyl | cis-DMM | O | $n_D^{47}$ : 1,5375 |
| 1.138. | 5-Chlor-2-pyridyl | trans-DMM | O | |
| 1.139. | 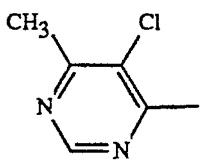 | DMM | O | |
| 1.140. | 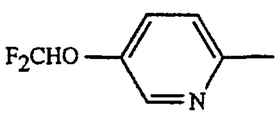 | Pip. | O | |
| 1.141. | 6-Methoxy-2-pyridyl | DMM | O | |
| 1.142. | 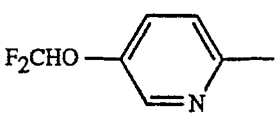 | DMM | O | |

| Nr. | Q | A | X | phys. Daten |
|---|---|---|---|---|
| 1.143. | 3-Pyridyl | Pip. | O | |
| 1.144. | 5-Chlor-2-pyridyl | 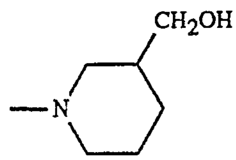 | O | |
| 1.145. | 5-Chlor-2-thienyl | trans-DMM | O | |
| 1.146. | 5-Chlor-2-thienyl | cis-DMM | O | |
| 1.147. | 5-Brom-2-pyridyl | 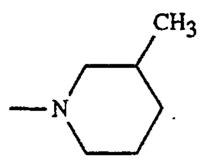 | O | |
| 1.148. | 4-Pyridyl | DMP | O | |
| 1.149. | 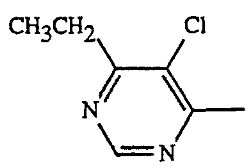 | trans-DMM | O | |
| 1.150. | 5-Chlor-2-pyridyl | 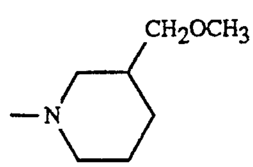 | O | |
| 1.151. | 3-Chlor-2-pyridyl | cis-DMM | O | |
| 1.152. | 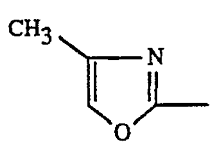 | DMM | O | |

| Nr. | Q | A | X | phys. Daten |
|---|---|---|---|---|
| 1.153. | 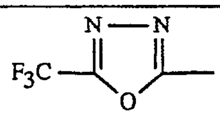 | DMM | O | |
| 1.154. | 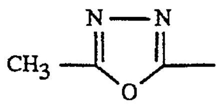 | Pip. | O | |
| 1.155. | 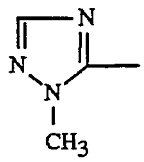 | DMM | O | |
| 1.156. | 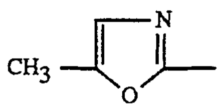 | DMM | O | |
| 1.157. | 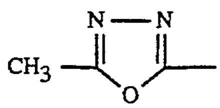 | DMM | O | |
| 1.158. | 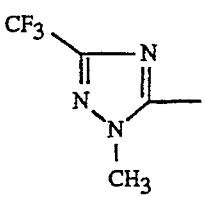 | DMM | O | |
| 1.159. | 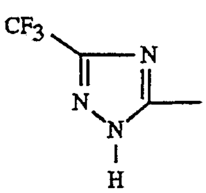 | DMM | O | |
| 1.160. | 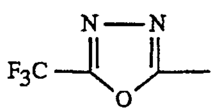 | Pip. | O | |

| Nr. | Q | A | X | phys. Daten |
|---|---|---|---|---|
| 1.161. | 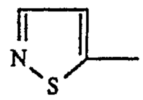 | DMM | O | |
| 1.162. | 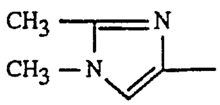 | DMM | O | |
| 1.163. | 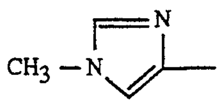 | DMM | O | |
| 1.164. | 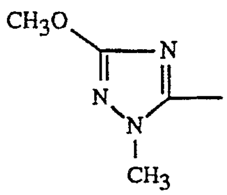 | DMM | O | |
| 1.165. | 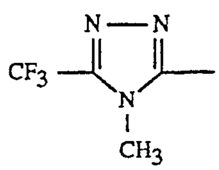 | DMM | O | |
| 1.166. | 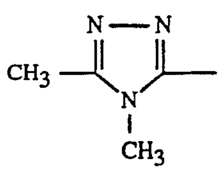 | DMM | O | |
| 1.167. | 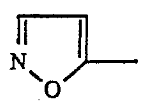 | DMM | O | |
| 1.168. | 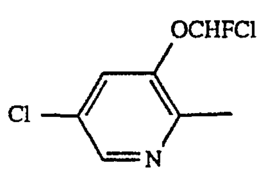 | cis-DMM | O | $n_D^{40}$ : 1,5245 |

| Nr. | Q | A | X | phys. Daten |
|---|---|---|---|---|
| 1.169. | 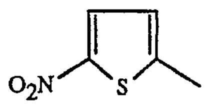 | cis-DMM | O | $n_D^{40}$ : 1,5632 |
| 1.170. | 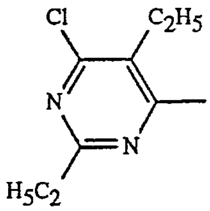 | cis-DMM | | $n_D^{24}$ : 1, 5318 |

Tabelle 2

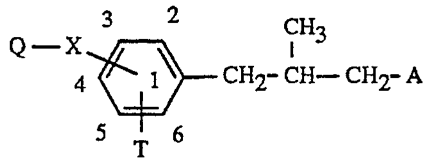

| Nr. | -X-Q | T | A | phys. Daten |
|---|---|---|---|---|
| 2.1 | 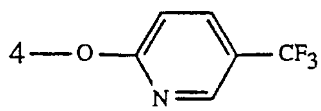 | - | DMM | |
| 2.2. | 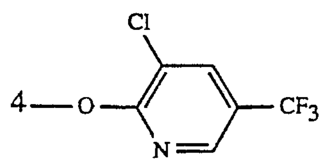 | - | Morph | |
| 2.3. | 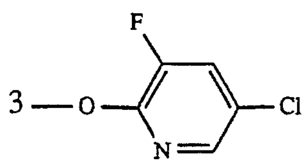 | 4-F | DMM | |
| 2.4. | 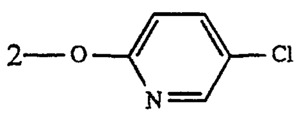 | - | Pip. | |
| 2.5. | 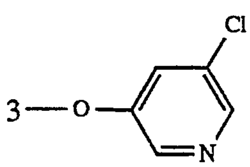 | - | DMP | |

| Nr. | -X-Q | T | A | phys. Daten |
|---|---|---|---|---|
| 2.6. | 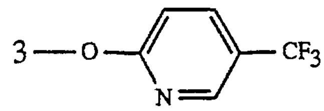 | 4-Cl | Pip. | |
| 2.7. | 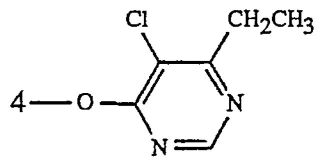 | - | DMM | |
| 2.8. | 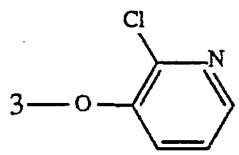 | - | Pip. | |
| 2.9. | 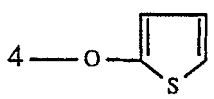 | - | DMM | |
| 2.10. | 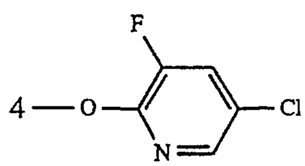 | 2-Cl | Pip. | |
| 2.11. | 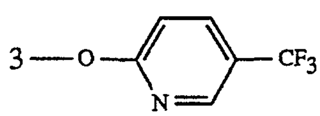 | 4-Cl | DMM | |
| 2.12. | 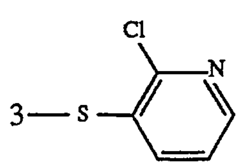 | - | DMM | |

| Nr. | -X-Q | T | A | phys. Daten |
|---|---|---|---|---|
| 2.13. | 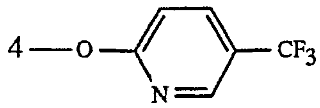 | - | Pip. | |
| 2.14. | 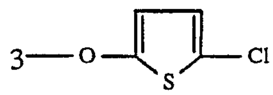 | 4-Cl | DMM | |
| 2.15. | 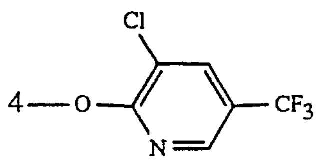 | - | DMM | |
| 2.16 | 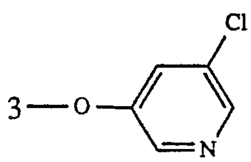 | - | Pip. | |
| 2.17. | 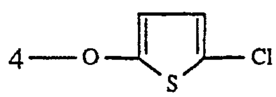 | - | DMM | |
| 2.18. | 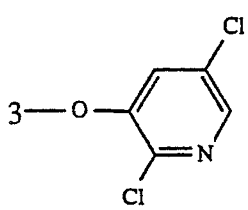 | 4-F | Pip. | |
| 2.19. | 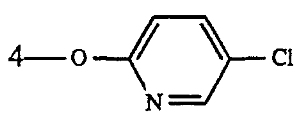 | - | DMM | |

| Nr. | -X-Q | T | A | phys. Daten |
|---|---|---|---|---|
| 2.20. | 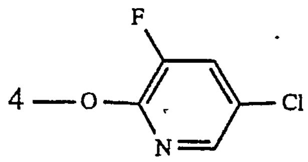 | 2-Cl | DMM | |
| 2.21. | 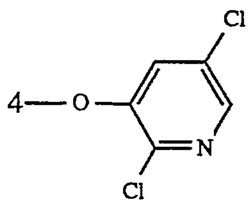 | - | Pip. | |
| 2.22. | 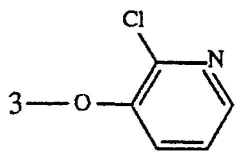 | - | DMP | |
| 2.23. | 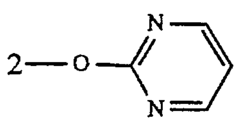 | - | DMM | |
| 2.24. | 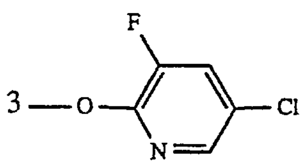 | 4-$CH_3$ | Pip. | |
| 2.25. | 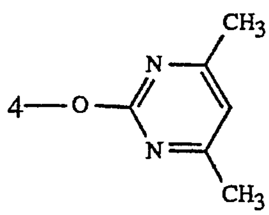 | - | DMM | |
| 2.26. | 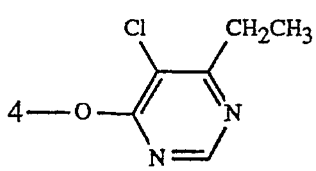 | - | Pip. | |

| Nr. | -X-Q | T | A | phys. Daten |
|---|---|---|---|---|
| 2.27. | 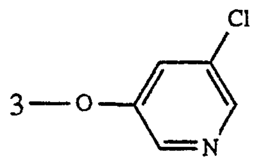 | - | DMM | |
| 2.28. | 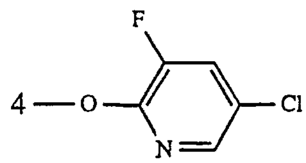 | - | Pip. | |
| 2.29. | 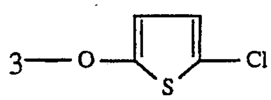 | 4-Cl | Pip. | |
| 2.30. | 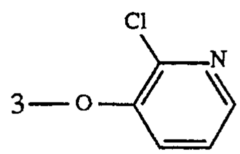 | - | trans-DMM | |
| 2.31 | 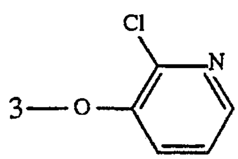 | - | cis-DMM | |
| 2.32. | 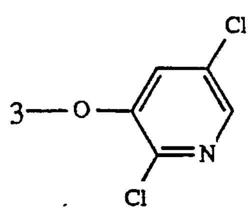 | 4-F | DMM | |
| 2.33. | 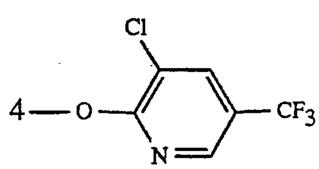 | - | Pip. | |

| Nr. | -X-Q | T | A | phys. Daten |
|---|---|---|---|---|
| 2.34. | 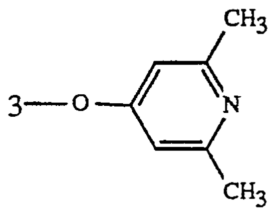 | - | DMM | |
| 2.35. | 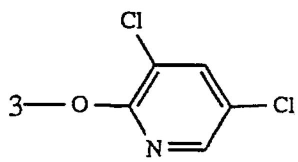 | 4-Cl | Pip. | |
| 2.36. | 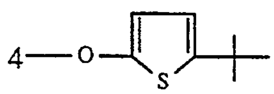 | - | Pip. | |
| 2.37. | 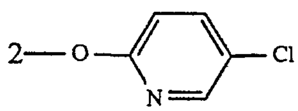 | - | DMM | |
| 2.38. | 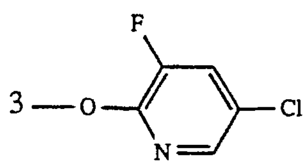 | 4-F | Pip. | |
| 2.39. | 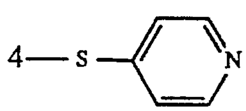 | - | DMM | |
| 2.40. | 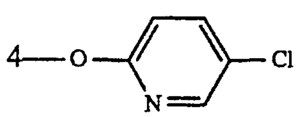 | - | Pip. | |
| 2.41. | 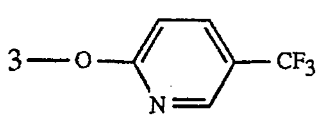 | $4\text{-}OCH_3$ | DMM | |

| Nr. | -X-Q | T | A | phys. Daten |
|---|---|---|---|---|
| 2.42. | 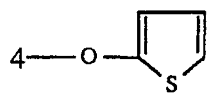 | - | Pip. | |
| 2.43. | 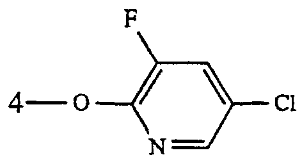 | - | DMM | |
| 2.44. | 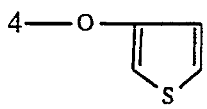 | - | DMM | |
| 2.45. | 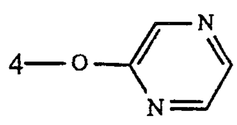 | - | Pip. | |
| 2.46. | 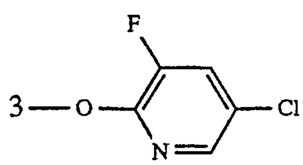 | 4-$CH_3$ | DMM | |
| 2.47. | 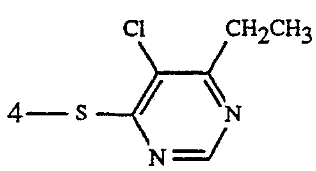 | - | DMM | |
| 2.48. | 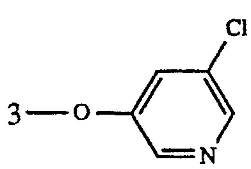 | - | cis-DMM | |

| Nr. | -X-Q | T | A | phys. Daten |
|---|---|---|---|---|
| 2.49. | 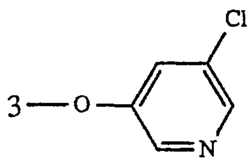 | - | trans-DMM | |
| 2.50. | 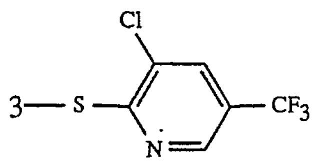 | 4-Cl | Pip. | |
| 2.51. | 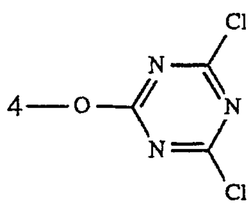 | - | DMM | |
| 2.52. | 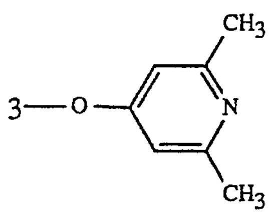 | - | Pip. | |
| 2.53. | 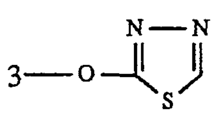 | - | DMM | |
| 2.54. | 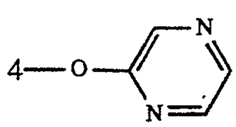 | - | DMM | |
| 2.55. | 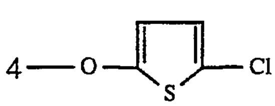 | - | Pip. | |

| Nr. | -X-Q | T | A | phys. Daten |
|---|---|---|---|---|
| 2.56. | 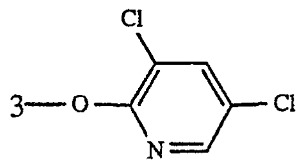 | 4-Cl | DMM | |
| 2.57. | 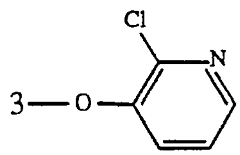 | - | DMM | |
| 2.58. | 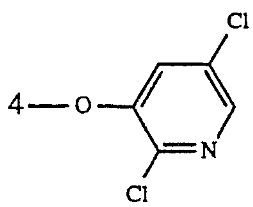 | - | DMM | |
| 2.59. | 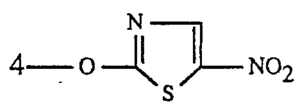 | - | DMM | |
| 2.60. | 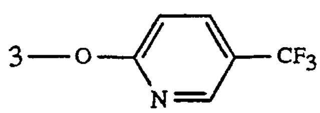 | 4-$OCH_3$ | Pip. | |
| 2.61. | 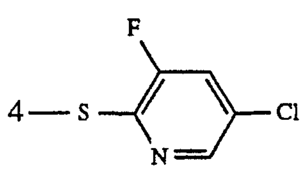 | - | DMM | |
| 2.62. | 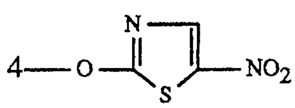 | - | Pip. | |

| Nr. | -X-Q | T | A | phys. Daten |
|---|---|---|---|---|
| 2.63. | 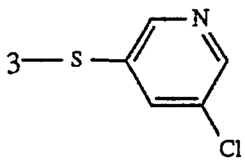 | - | DMM | |
| 2.64. | 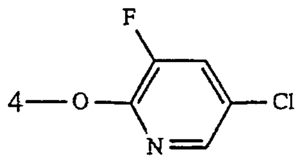 | - | trans-DMM | |
| 2.65. | 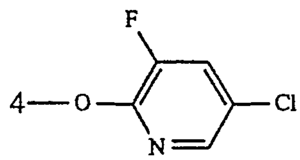 | - | cis-DMM | |
| 2.66. | 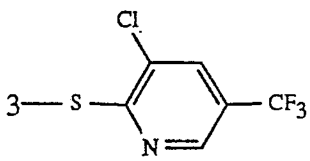 | 4-Cl | DMM | |
| 2.67. | 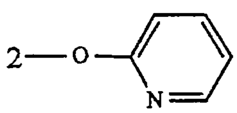 | - | DMM | |
| 2.68. | 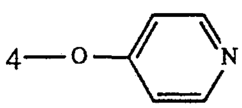 | - | DMM | |
| 2.69. | 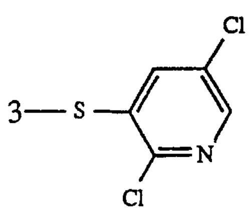 | - | DMM | |

| Nr. | -X-Q | T | A | phys. Daten |
|---|---|---|---|---|
| 2.70. | 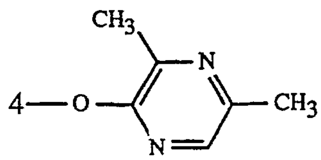 | - | DMM | |
| 2.71. | 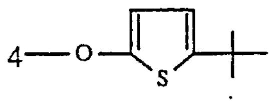 | - | DMM | |
| 2.72. | 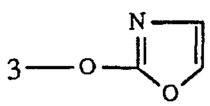 | - | Pip. | |
| 2.73. | 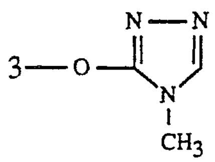 | - | DMM | |

Tabelle 3

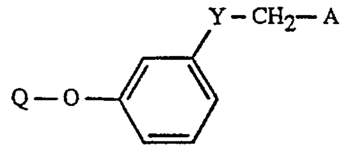

| Nr. | Q | Y | A | phys. Daten |
|---|---|---|---|---|
| 3.1. | 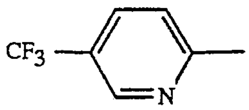 | $-CH_2$ | DMM | |
| 3.2. | 3-Pyridazinyl | 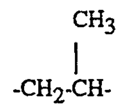 | Pip. | |
| 3.3. | 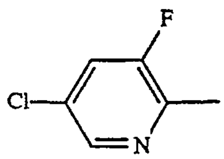 | 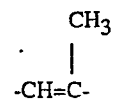 | DMM | |
| 3.4. | 2,5-Dichlor-3-pyridyl | 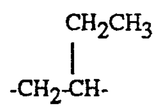 | Pip. | |
| 3.5. | 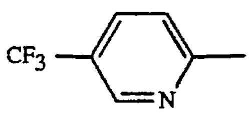 | -CH=CH- | DMM | |
| 3.6. | 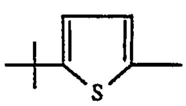 | 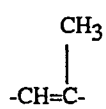 | Pip. | |
| 3.7. | 2-Pyrimidinyl | $-CH_2CH_2-$ | DMM | |
| 3.8. | 3,5-Dichlor-2-pyridyl | 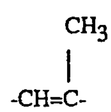 | Morph | |

| Nr. | Q | Y | A | phys. Daten |
|---|---|---|---|---|
| 3.9. | 3,5-Dichlor-2-pyridyl | $CH_3$<br>\|<br>-CH=C- | DMM | |
| 3.10. | 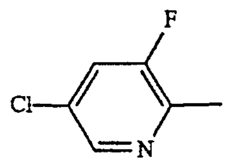 | $CH_3$<br>\|<br>-CH=C- | Pip. | |
| 3.11. | 5-Chlor-2-thiazolyl | $CH_3$<br>\|<br>-CH=C- | DMM | |
| 3.12. | 2,5-Dichlor-3-pyridyl | $CH_2CH_3$<br>\|<br>$-CH_2-CH-$ | DMM | |
| 3.13. | 2-Pyrazinyl | $CH_3$<br>\|<br>-CH=C- | DMM | |
| 3.14. | 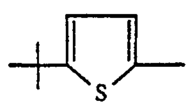 | $CH_3$<br>\|<br>-CH=C- | DMM | |
| 3.15. | 5-Chlor-2-pyridyl | $CH_3$<br>\|<br>-CH=C- | Pip. | |
| 3.16. | 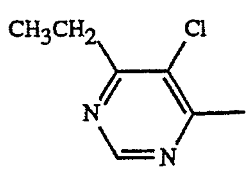 | $CH_3$<br>\|<br>-CH=C- | DMM | |
| 3.17. | 5-Chlor-2-pyridyl | $CH_3$<br>\|<br>-CH=C- | DMM | |

| Nr. | Q | Y | A | phys. Daten |
|---|---|---|---|---|
| 3.18. | 2-Oxazolyl | 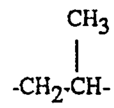 | DMM | |
| 3.19. | 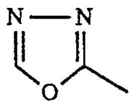 | 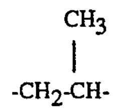 | DMM | |
| 3.20. | 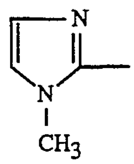 | 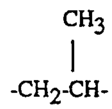 | Pip. | |
| 3.21. | 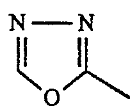 | 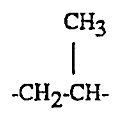 | Pip. | |

Formulierungsbeispiele für Wirkstoffe der Formel I

(% = Gewichtsprozent)

| F1. Lösungen | a) | b) | c) | d) |
|---|---|---|---|---|
| Wirkstoff aus den Tabellen | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190 °C) | - | - | 94 % | - |
| (MG = Molekulargewicht) | | | | |

Die Lösungen sind zur Anwendung in Form kleinster Tropfen geeignet.

| F2. Granulate | a) | b) |
|---|---|---|
| Wirkstoff aus den Tabellen | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| F3. Stäubemittel | a) | b) |
|---|---|---|
| Wirkstoff aus den Tabellen | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

| F4. Spritzpulver | a) | b) | c) |
|---|---|---|---|
| Wirkstoff aus den Tabellen | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| F5. Emulsions-Konzentrat | |
|---|---|
| Wirkstoff aus den Tabellen | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| F6. Umhüllungs-Granulat | |
|---|---|
| Wirkstoff aus den Tabellen | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

Biologische Beispiele:

Beispiel B1: Wirkung gegen Puccinia graminis auf Weizen

a) Residual-protektive Wirkung

Weizenpflanzen werden 6 Tage nach der Aussaat mit einer aus Spritzpulver des Wirkstoffes hergestellten Spritzbrühe (0,02 % Aktivsubstanz) besprüht. Nach 24 Stunden werden die behandelten Pflanzen mit einer Uredosporensuspension des Pilzes infiziert. Nach einer Inkubation während 48 Stunden bei 95-100 % relativer Luftfeuchtigkeit und ca. 20 °C werden die infizierten Pflanzen in einem Gewächshaus bei ca. 22 °C aufgestellt. Die Beurteilung der Rostpustelnentwicklung erfolgt 12 Tage nach der Infektion.

b) Systemische Wirkung

Zu Weizenpflanzen wird 5 Tage nach der Aussaat eine aus Spritzpulver des Wirkstoffes hergestellte Spritzbrühe gegossen (0,006 % Aktivsubstanz bezogen auf das Bodenvolumen). Nach 48 Stunden werden die behandelten Pflanzen mit einer Uredosporensuspension des Pilzes infiziert. Nach einer Inkubation während 48 Stunden bei 95-100 % relativer Luftfeuchtigkeit und ca. 20 °C werden die infizierten Pflanzen in einem Gewächshaus bei ca. 22 °C aufgestellt. Die Beurteilung der Rostpustelnentwicklung erfolgt 12 Tage nach der Infektion.

Unbehandelte aber infizierte Kontrollpflanzen zeigten einen Puccinia-Befall von 100 % Verbindungen aus den Tabellen zeigten gegen Puccinia-Pilze eine gute Wirkung (5-20 % Befall). Die Verbindungen Nr. 1.1, 1.2a, 1.2b, 1.10, 1.13, 1.14, 1.28, 1.29, 1.31, 1.36, 1.39, 1.42, 1.51, 1.61, 1.75, 1.87, 1.91, 1.105, 1.168, 1.170 und andere hemmtenden Befall auf 0.5%.

Beispiel B2: Wirkung gegen Cercospora arachidicola auf Erdnusspflanzen

a) Residual-protektive Wirkung

10-15 cm hohe Erdnusspflanzen werden mit einer aus Spritzpulver der Wirksubstanz hergestellten Spritzbrühe (0,02 % Aktivsubstanz) besprüht und 48 Stunden später mit einer Konidiensuspension des Pilzes infiziert. Die infizierten Pflanzen werden während 72 Stunden bei ca. 21 °C und hoher Luftfeuchtigkeit inkubiert und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

b) Systemische Wirkung

Zu 10-15 cm hohen Erdnusspflanzen wird eine aus Spritzpulver des Wirkstoffes hergestellte Spritzbrühe gegossen (0,06 % Aktivsubstanz bezogen auf das Erdvolumen). Nach 48 Stunden werden die behandelten Pflanzen mit einer Konidiensuspension des Pilzes infiziert und 72 Stunden bei ca. 21 °C und hoher Luftfeuchigkeit inkubiert. Anschliessend werden die Pflanzen im Gewächshaus aufgestellt, und nach 11 Tagen wird der Pilzbefall beurteilt.

Im Vergleich zu unbehandelten, aber infizierten Kontrollpflanzen (Anzahl und Grösse der Flecken = 100%), zeigten Erdnusspflanzen, die mit Wirkstoffen aus den Tabellen behandelt wurden, einen stark reduzierten Cercospora-Befall. So verhinderten die Verbindungen Nr. 1.1, 1.6, 1.10, 1.12, 1.13, 1.14, 1.22, 1.24, 1.28, 1.29, 1.31, 1.32, 1.33, 1.36, 1.39, 1.40, 1.42, 1.43, 1.51, 1.57, 1.58, 1.61, 1.75, 1.82, 1.87, 1.91, 1.105, 1.113, 1.124, 1.126, 1.140, 1.143, 1.151, 1.168, 1.169, 1.170, 2.1, 2.2, 2.17, 2.62, 2.70, 2.71 und 3.2 in obigen Versuchen das Auftreten von Flecken fast vollständig (0-10 %).

Beispiel B3: Wirkung gegen Erysiphae graminis auf Gerste

a) Residual-protektive Wirkung

Ca. 8 cm hohe Gerstenpflanzen werden mit einer aus Spritzpulver des Wirkstoffes hergestellten Spritzbrühe (0,02 % Aktivsubstanz) besprüht. Nach 3-4 Stunden werden die behandelten Pflanzen mit Konidien des Pilzes bestäubt. Die infizierten Gerstenpflanzen werden in einem Gewächshaus bei ca. 22 °C aufgestellt, und der Pilzbefall wird nach 10 Tagen beurteilt.

b) Systemische Wirkung

Zu ca. 8 cm hohen Gerstenpflanzen wird eine aus Spritzpulver des Wirkstoffes hergestellte Spritzbrühe gegossen (0,006 % Aktivsubstanz bezogen auf das Erdvolumen). Es wird dabei darauf geachtet, dass die Spritzbrühe nicht mit den oberirdischen Pflanzenteilen in Berührung kommt. Nach 48 Stunden werden die behandelten Pflanzen mit Konidien des Pilzes bestäubt. Die infizierten Gerstenpflanzen werden in einem Gewächshaus bei ca. 22 °C aufgestellt und der Pilzbefall nach 10 Tagen beurteilt.

Verbindungen der Formel I zeigten eine gute Wirkung gegen Erysiphae-Pilze. Unbehandelte, aber infizierte Kontrollpflanzen zeigten einen Erysiphae-Befall von 100 %. Unter anderen Verbindungen aus den Tabellen hemmten die Verbindungen Nr. 1.1,1 .2a, 1.2b, 1.6, 1.14, 1.20, 1.24, 1.27, 1.28, 1.29, 1.36, 1.40, 1.43, 1.44, 1.61, 1.168, 1.169, 1.170, 2.5, 2.13, 2.14, 21.5, 2.20, 2.24, 2.41, 2.42, 3.1, 3.2, 3.19 den Pilzbefall bei Gerste auf 0 bis 5%.

Beispiel B4: Residual-protektive Wirkung gegen Venturia inaequalis auf Apfeltrieben

Apfelstecklinge mit 10-20 cm langen Frischtrieben werden mit einer aus Spritzpulver des Wirkstoffes hergestellten Spritzbrühe (0,06 % Aktivsubstanz) besprüht. Nach 24 Stunden werden die behandelten Pflanzen mit einer Konidiensuspension des Pilzes infiziert. Die Pflanzen werden dann während 5 Tagen bei 90-100% relativer Luftfeuchtigkeit inkubiert und während 10 weiteren Tagen in einem Gewächshaus bei 20-24 °C aufgestellt. Der Schorfbefall wird 15 Tage nach der Infektion beurteilt. Verbindungen aus den Tabellen bewirkten eine deutliche Hemmung des Krankheitsbefalls. So hemmten die Verbindungen 1.1, 1.2a, 1.2b, 1.24, 1.28, 1.40, 1.43, 1.168, 1.170, 2.15, 2.20, 2.24 und 3.2 den Pilzbefall fast vollständig (0.5 %). Unbehandelte aber infizierte Triebe zeigten einen 100%igen Venturia-Befall.

Beispiel B5: Wirkung gegen Botrytis cinerea auf Bohnen

Residual protektive Wirkung

Ca. 10cm hohe Bohnen-Pflanzen werden mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infiziert. Nach einer Inkubation der infizierten Pflanzen während 3 Tagen bei 95-100 % relativer Luftfeuchtigkeit und 21 °C erfolgt die Beurteilung des Pilzbefalls.

Der Botrytis-Befall unbehandelter aber infizierter Bohnenpflanzen betrug 100 %. Der Befall nach Behandlung mit einer der Verbindungen der Formel I betrug <20 %; bei Behandlung mit den Verbindungen Nr. 1.1, 1.2, 1.10, 1.14, 1.19, 1.22, 1.24, 1.26, 1.28, 1.32, 1.39, 1.51, 1.66, 1.78, 1.82, 1.93, 1.99, 1.120, 1.124, 1.152, 1.157, 1.158, 1.163, 1.165, 1.168, 1.170, 2.1, 2.9, 2.14, 2.25, 2.53, 2.59, 3.3, 3.14 und anderen trat kein Befall auf (0-5%).

Beispiel B6: Wirkung gegen Rhizoctonia solani (Bodenpilz auf Reispflanzen)

a) Protektiv-lokale Bodenapplikation

12 Tage alte Reispflanzen werden mit einer aus einer Formulierung des Wirkstoffes hergestellten Spritzbrühe (0,006 % Aktivsubstanz), ohne oberirdische Pflanzenteile zu benetzen, angegossen. Zur Infizierung der behandelten Pflanzen wird eine Suspension von Myzel und Sklerotien von R. Solani auf die Bodenoberfläche gegeben. Nach 6-tägiger Inkubation bei 27 °C (Tag), bzw. 23 ° (Nacht) und 100 % rel. Luftfeuchtigkeit (Feuchtkasten) in der Klimakammer wird der Pilzbefall auf Blattscheide, Blättern und

Stengel beurteilt.

b) Protektiv-lokale Blattapplikation

12 Tage alte Reispflanzen werden mit einer aus einer Formulierung der Wirkstoffe hergestellten Spritzbrühe besprüht. Einen Tag später werden die behandelten Pflanzen mit einer Suspension von Myzel und Sklerotien von R. solani infiziert. Nach 6-tägiger Inkubation bei 27° C (Tag), bzw. 23° C (Nacht) und 100 % rel. Luftfeuchtigkeit (Feuchtkasten) in der Klimakammer wird der Pilzbefall auf Blattscheide, Blättern und Stengel beurteilt.

Verbindungen aus den Tabellen zeigten gute Wirksamkeit durch Hemmung des Rhizoctonia-Befalls. So hemmten z.B. die Verbindungen 1.1, 1.14, 1.16, 1.17, 1.22, 1.28, 1.39, 1.82, 1.168, 2.1, 2.3, 2.4, 2.14, 2.17, 3.1, 3.3 den Pilzbefall bis auf 0 bis 5 %. Unbehandelte jedoch infizierte Kontrollpflanzen wiesen dagegen einen Befall von 100 % auf.

## Ansprüche

1. Verbindungen der Formel I

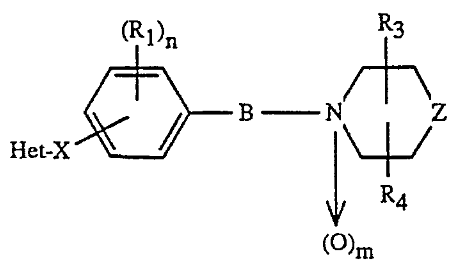

(I)

worin bedeuten:
X Sauerstoff oder Schwefel,
Het einen 5- oder 6-gliedrigen Heterocyclus, mit einem bis drei gleichen oder verschiedenen Heteroatomen ausgewählt aus Stickstoff, Sauerstoff und Schwefel, der unsubstituiert ist oder mit gleichen oder verschiedenen Substituenten ausgewählt aus Halogen, Cyano, Nitro, $C_1$-$C_4$-Alkyl, $C_3$-$C_6$-Cycloalkyl, $C_1$-$C_4$-Alkoxy, $C_1$-$C_3$-Haloalkoxy und Trifluormethyl ein-, zwei- oder dreifach substituiert ist,
B $-CH_2-CH(R_2)-CH_2-$ oder $-CH=C(R_2)-CH_2-$ oder $-CH_2-C(R_2)=CH-$,
$R_1$ Halogen, $C_1$-$C_4$-Alkyl, $C_1$-$C_4$-Alkoxy,
n 0 bis 2,
m 0 oder 1,
$R_2$ Wasserstoff, $C_1$-$C_4$-Alkyl,
$R_3$ Wasserstoff, $C_1$-$C_3$-Alkyl,
$R_4$ Wasserstoff, Hydroxy, $C_1$-$C_3$-Alkoxy oder aber $C_1$-$C_4$-Alkyl, das unsubstituiert oder durch Hydroxy oder $C_1$-$C_3$-Alkoxy substituiert ist,
Z Sauerstoff, Schwefel oder $-CH_2-$;
unter Einschluss der Säureadditionssalze dieser Verbindungen.

2. Verbindungen gemäss Anspruch 1, worin der 5- oder 6-gliedrige Heterocyclus Het mindestens ein N-Atom enthält, $R_1$ Halogen, Methyl oder Methoxy bedeutet, n 0 oder 1 ist, m 0 ist und $R_2$ und $R_3$ unabhängig voneinander Wasserstoff oder Methyl bedeuten.

3. Verbindungen gemäss Anspruch 2, worin X Sauerstoff bedeutet und $R_4$ Wasserstoff, Hydroxy, Methyl, Methoxy, Hydroxymethyl, Hydroxyethyl, Methoxymethyl oder Methoxyethyl darstellt.

4. Verbindungen gemäss Anspruch 3, worin Het einen unsubstituierten oder substituierten Pyridylrest bedeutet.

5. Verbindungen gemäss Anspruch 4, worin B die Kette $-CH_2-CH(CH_3)-CH_2-$ oder $-CH=C(CH_3)-CH_2-$ bedeutet, und der Pyridylrest unsubstituiert oder durch maximal zwei der Substituenten Halogen, $NO_2$, $-OCHF_2$ und $CF_3$ gleich oder verschieden substituiert ist.

6. Verbindungen gemäss Anspruch 5, worin Z Sauerstoff oder $-CH_2-$ darstellt und $R_3$ und $R_4$ die beiden zu Z benachbarten Stellungen einnehmen und beide die gleiche Bedeutung Wasserstoff oder Methyl haben.

7. Verbindungen gemäss Anspruch 3, worin Het einen unsubstituierten oder substituierten Pyrimidinylrest bedeutet.

8. Verbindungen gemäss Anspruch 7, worin B die Kette $-CH_2-CH(CH_3)-CH_2-$ oder $-CH=C(CH_3)-CH_2-$ bedeutet, und der Pyrimidinylrest unsubstituiert ist oder durch Halogen, $C_1$-$C_4$-Alkyl, Cyclopropyl gleich oder verschieden ein- bis dreifach substituiert ist.

9. Verbindungen gemäss Anspruch 8, worin Z Sauerstoff oder $-CH_2-$ darstellt und $R_3$ und $R_4$ die beiden zu Z benachbarten Stellungen einnehmen und beide die gleiche Bedeutung Wasserstoff oder Methyl haben.

10. Verbindungen gemäss Anspruch 3, worin Het einen unsubstituierten oder substituierten Pyrazinylrest bedeutet.

11. Verbindungen gemäss Anspruch 10, worin B die Kette $-CH_2-CH(CH_3)-CH_2-$ oder $-CH=C(CH_3)-CH_2-$ bedeutet und der Pyrazinylrest unsubstituiert oder durch $C_1$-$C_4$-Alkyl und/oder Halogen maximal zweifach substituiert ist.

12. Verbindungen gemäss Anspruch 11, worin Z Sauerstoff oder $-CH_2-$ darstellt und $R_3$ und $R_4$ die beiden zu Z benachbarten Stellungen einnehmen und beide die gleiche Bedeutung Wasserstoff oder Methyl haben.

13. Verbindungen gemäss Anspruch 3, worin Het einen unsubstituierten oder substituierten 1,3,5-Triazinylrest bedeutet.

14. Verbindungen gemäss Anspruch 13, worin B die Kette $-CH_2-CH(CH_3)-CH_2-$ oder $-CH=C(CH_3)-CH_2-$ bedeutet und der 1,3,5-Triazinylrest unsubstituiert, alkyl- und/oder halogensubstituiert ist.

15. Verbindungen gemäss Anspruch 14, worin Z Sauerstoff oder $-CH_2-$ darstellt und $R_3$ und $R_4$ die beiden zu Z benachbarten Stellungen einnehmen und beide die gleiche Bedeutung Wasserstoff oder Methyl haben.

16. Verbindungen gemäss Anspruch 3, worin Het einen unsubstituierten oder substituierten Thiazolylrest bedeutet.

17. Verbindungen gemäss Anspruch 16, worin B die Kette $-CH_2-CH(CH_3)-CH_2-$ oder $-CH=C(CH_3)-CH_2-$ bedeutet, und der Thiazolylrest unsubstituiert oder durch $NO_2$ oder Halogen monosubstituiert ist.

18. Verbindungen gemäss Anspruch 17, worin Z Sauerstoff oder $-CH_2-$ darstellt und $R_3$ und $R_4$ die beiden zu Z benachbarten Stellungen einnehmen und beide die gleiche Bedeutung Wasserstoff oder Methyl haben.

19. Verbindungen gemäss Anspruch 3, worin Het einen Oxazolylrest bedeutet, Z Sauerstoff oder $-CH_2-$ darstellt und $R_3$ und $R_4$ die beiden zu Z benachbarten Stellungen einnehmen und beide die gleiche Bedeutung Wasserstoff oder Methyl haben.

20. Verbindungen gemäss Anspruch 3, worin Het einen unsubstituierten oder durch $C_1$-$C_4$-Alkyl substituierten 1,2,4-Triazolylrest bedeutet, Z Sauerstoff oder $-CH_2-$ darstellt und $R_3$ und $R_4$ die beiden zu Z benachbarten Stellungen einnehmen und beide die gleiche Bedeutung Wasserstoff oder Methyl haben.

21. Verbindungen gemäss Anspruch 3, worin Het einen unsubstitierten oder durch $C_1$-$C_4$-Alkyl substituierten 1,2,4-Imidazolylrest bedeutet, Z Sauerstoff oder $-CH_2-$ darstellt und $R_3$ und $R_4$ die beiden zu Z benachbarten Stellungen einnehmen und beide die gleiche Bedeutung Wasserstoff oder Methyl haben.

22. Verbindungen gemäss Anspruch 3, worin Het einen Oxadiazolrest bedeutet, Z Sauerstoff oder $-CH_2-$ darstellt und $R_3$ und $R_4$ die beiden zu Z benachbarten Stellungen einnehmen und beide die gleiche Bedeutung Wasserstoff oder Methyl haben.

23. Verbindungen gemäss Anspruch 3, worin Het einen Thiadiazolrest bedeutet, Z Sauerstoff oder $-CH_2-$ darstellt und $R_3$ und $R_4$ die beiden zu Z benachbarten Stellungen einnehmen und beide die gleiche Bedeutung Wasserstoff oder Methyl haben.

24. Verbindungen gemäss Anspruch 1, worin Het einen unsubstituierten oder durch Halogen, oder $C_1$-$C_4$Alkyl gleich oder verschieden maximal zweifach substituierten Thienylrest bedeutet.

25. Verbindungen gemäss Anspruch 24, worin X Sauerstoff bedeutet, n 0 oder 1 ist, m 0 ist, $R_1$ Halogen, Methyl oder Methoxy darstellt, $R_2$ Wasserstoff oder Methyl und $R_3$ Wasserstoff oder Methyl bedeuten, und $R_4$ Wasserstoff, Methyl, Hydroxy, Methoxy, Hydroxymethyl oder Methoxymethyl darstellt.

26. Verbindungen gemäss Anspruch 25, worin Z Sauerstoff oder $-CH_2-$ darstellt und $R_3$ und $R_4$ die beiden zu Z benachbarten Stellungen einnehmen und beide die gleiche Bedeutung Wasserstoff oder Methyl haben.

27. Verfahren zur Herstellung von Verbindungen der Formel I gemäss Anspruch 1, gekennzeichnet

a) durch Reaktion eines Amins der Formel II

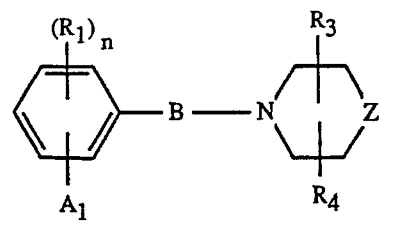

(II)

bei 0° bis 220° C mit einem Heterocyclus der Formel III

Het-$A_2$ (III)

worin einer der Reste $A_1$ und $A_2$ eine nukleofuge Abgangsgruppe und der andere die Gruppe -XMe bedeutet, in der Me Wasserstoff oder ein Metallkation ist, während die Substituenten $R_1$, $R_3$, $R_4$, B, X und Z sowie n die unter Formel I angegebenen Bedeutungen haben; oder

b) durch Kondensation eines heterocyclischen Diphenylethers der Formel IV

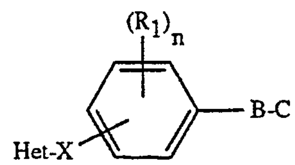

(IV)

worin C eine nukleofuge Abgangsgruppe darstellt, bei 0° bis 120° C mit einem Amin der Formel V

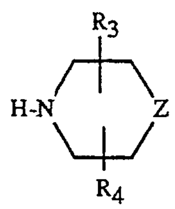

(V)

wobei die Substituenten $R_1$, $R_3$, $R_4$, B, X, Het und Z sowie n die unter Formel I angegebenen Bedeutungen haben; oder

c) sofern B die Kette $-CH_2-C(R_2)=CH-$ oder $-CH_2-CH(R_2)-CH_2-$ bedeutet, durch Reaktion eines Aldehyds der Formel VI

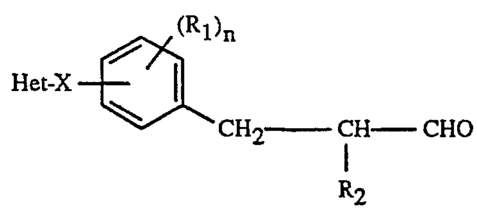

(VI)

bei -25° C bis +300° C, mit einem Amin der Formel V zu einer Verbindung der Formel VII

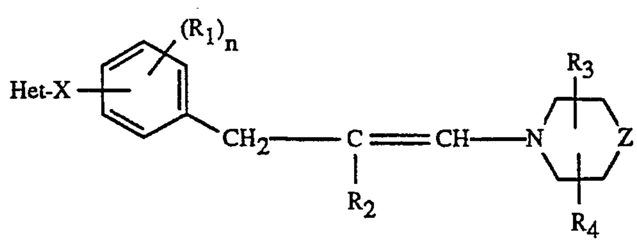

(VII)

im Umfang der Formel I und, sofern gewünscht, anschliessend Hydrierung der Doppelbindung, wobei die Substituenten $R_1$, $R_2$, $R_3$, $R_4$, X, Het, n und Z die unter Formel I gegebenen Bedeutungen haben; unter gegebenenfalls weiterer Oxidation der so erhaltenen Verbindungen der Formel I mit $m=0$ zur Erzielung der entsprechenden N-Oxide ($m=1$) der Formel I.

28. Mikrobizide Mittel enthaltend als mindestens einen Wirkstoff eine Verbindung der Formel I gemäss Anspruch 1, zusammen mit einem geeigneten Trägermaterial.

29. Mittel gemäss Anspruch 28 enthaltend als Wirkstoff eine Verbindung gemäss einem der Ansprüche 2 bis 26.

30. Verwendung von Verbindungen der Formel I gemäss einem der Ansprüche 1 bis 26 zur Bekämpfung

oder Verhütung eines Befalls von phytopathogenen Mikroorganismen.

31. Verfahren zur Bekämpfung oder Verhütung eines Befalls von Pflanzen durch phytopathogene Mikroorganismen, dadurch gekennzeichnet, dass man eine Verbindung der Formel I gemäss einem der Ansprüche 1 bis 26 auf die Pflanze, Pflanzenteile oder deren Standort appliziert.

32. Verfahren gemäss Anspruch 31, wobei die Pflanzenteile das Saatgut sind.

Europäisches Patentamt

# EUROPÄISCHER RECHERCHENBERICHT

Nummer der Anmeldung

EP 90112118.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| P,X | CHEMICAL ABSTRACTS, Band 112, Nr. 19, 7. Mai 1990 Columbus, Ohio, USA MEKI NAOTO et al. "Morpholines as agrochemical fungicides" Seite 773, Spalte 2, Zusammenfassung-Nr. 179 000f & Jpn. Kokai Tokkyo Koho JP 01,254,680 (89,254,680) 11 Oct. 1989 -- | 1-32 | C 07 D 401/12<br>C 07 D 403/12<br>C 07 D 405/12<br>C 07 D 409/12<br>C 07 D 413/12<br>C 07 D 417/12<br>A 01 N 43/40<br>A 01 N 43/84 |
| A | EP - A2 - 0 262 870 (SUMITOMO) * Ansprüche 1,12 * -- | 1,28 | |
| D,A | DE - A1 - 2 752 135 (HOFFMANN) * Ansprüche 1,12 * ---- | 1,28 | |

RECHERCHIERTE SACHGEBIETE (Int Cl 5)

C 07 D 401/00
C 07 D 403/00
C 07 D 405/00
C 07 D 409/00
C 07 D 413/00
C 07 D 417/00

Der vorliegende Recherchenbericht wurde für alle Patentansprüche erstellt

| Recherchenort | Abschlußdatum der Recherche | Prüfer |
|---|---|---|
| WIEN | 18-09-1990 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82